(19) Europäisches Patentamt
European Patent Office
Office européen des brevets

(11) **EP 3 372 157 A1**

(12) **EUROPEAN PATENT APPLICATION**

(43) Date of publication:
**12.09.2018 Bulletin 2018/37**

(51) Int Cl.:
***A61B 5/0478*** (2006.01)     ***A61B 5/00*** (2006.01)

(21) Application number: **17159382.5**

(22) Date of filing: **06.03.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Mybrain Technologies**
**75010 Paris (FR)**

(72) Inventors:
• **ATTAL, Yohan**
**75010 Paris (FR)**

• **VANDENDRIESSCHE, Martin**
**75010 Paris (FR)**
• **POURCHIER, Nicolas**
**75010 Paris (FR)**
• **PASCAUD, Gatien**
**36000 Châteauroux (FR)**
• **RIVAUD, Valentin**
**75003 Paris (FR)**

(74) Representative: **Icosa**
**83 avenue Denfert-Rochereau**
**75014 Paris (FR)**

(54) **ELECTRODE SYSTEM FOR BIO-SIGNAL ACQUISITION**

(57)     The present invention relates to an electrode system (1) comprising:
- at least one flexible band (11);
- at least one movable electrode module (13) having at least one degree of freedom with reference to said at least one flexible band (11) and positionable at different locations on the at least one flexible band (11);
- at least one conductive cable connected to the at least one electrode module (13); and
- at least one electrode configured to fit in one movable electrode module (13), to be connected with the at least one conductive cable and to detect at least one bio-signal.

FIG. 1

EP 3 372 157 A1

**Description**

**FIELD OF INVENTION**

The present invention pertains to the field of bio-signal acquisition. In particular, the invention relates to an electrode system for positioning electrodes about the head of a subject to measure EEG signals.

**BACKGROUND OF INVENTION**

The study of the electrical activity of a subject's brain is essential in the diagnosis and the treatment of various diseases and conditions such as for example epilepsy, syncope, migraines, consciousness and alertness disorders (coma, confusion...), sleep disorders, encephalopathy, Creutzfeldt-Jakob disease, and cerebral lesions. It is also used to monitor the health of premature children, or to serve as an adjunct test of brain death.

The Electroencephalography (EEG) is the main method used to monitor and record the electrical activity of a brain. Typically noninvasive, the EEG consists in placing electrodes along the scalp of a subject and measures voltage fluctuations resulting from ionic current within the neurons of the brain. More particularly, the EEG records the brain's spontaneous electrical activity over a period of time. EEG systems are now increasingly used in non-clinical conditions, thus should be adaptable to various states of the subject, such as for example: lying down, sitting, or standing, unconscious, or keeping up with his daily activities.

However, the positioning and removal of the electrodes requires an operator with knowledge and skill to properly place and attach said electrodes to the subject skull. Moreover, this process is time-consuming and is often a source of discomfort for the subject.

The US patent application 2007/225,585 discloses an electrode headset comprising stretchable bands with voids therebetween, electrode modules included within the plurality of bands configured to receive an electrode, and an electronic circuitry configured to receive signals from said electrodes. However, such system is only adaptable to a limited number of predetermined electrode positions on the head of a subject, making the headset difficult to adjust to any size of head without losing the required electrode positions according to the measuring system 10/20.

The US patent 8,463,354 discloses an electrode system comprising a flexible circuit substrate; a plurality of removable electrode modules positionable at different predetermined locations of said substrate; circuit members layered against the substrate; removable electrodes disposed in each electrode modules; and a control box. The electrode modules are coupled with each other via flexible connectors. However, such system comprises non-dry electrodes making the electrode system fastidious to operate and to clean. Indeed, the use of non-dry electrodes requires to fill any gap between the skin and said electrode with a conductive gel or a saline solution, this step is time consuming and requires a qualified operator. Furthermore, the use of non-dry electrodes often leads to a bad conduction between the electrodes and the skin of the subject with various interference and impedance problems causing a low signal to noise ratio. In the electrode system disclosed in US 8,463,354, the flexible connectors reaching the electrode modules two by two are not layered to the substrate, this could increase the risk of involuntary removal of said connectors and be a cause of discomfort for the subject. Finally, such system is not adaptable to a wide range of electrode positions on the head of a subject as the positions of the electrode modules are limited and predetermined.

It is therefore an object of the present invention to provide an electrode system having one or more of the following advantages: easy to operate (positioning and removal), easy to clean, allowing the skin to breathe, easy to adjust to any size of head, adaptable to a wide range of electrode positions on the head of a subject, allowing a good conduction between the electrodes and the skin of the subject, appropriate for a clinical and/or non-clinical use, autonomous, compact with limited disorder due to conductive cables, thus preventing any discomfort or difficulties in the case of a subject already heavily medically equipped.

**SUMMARY**

The present invention relates to an electrode system comprising:

- at least one flexible band;
- at least one movable electrode module having at least one degree of freedom with reference to said at least one flexible band and positionable at different locations on the at least one flexible band;
- at least one conductive cable connected to the at least one movable electrode module; and
- at least one electrode configured to fit in one movable electrode module, to be connected with the at least one conductive cable and to detect at least one bio-signal.

According to one embodiment, the at least one movable electrode module comprises a lower part and an upper part configured to couple together in a first position and in a second position, the at least one flexible band passes between said lower and upper parts, and is retained in the first position or free to move in the second position. According to one embodiment, the at least one flexible band is a network of flexible bands. According to one embodiment, the flexible bands network comprises at least one first band configured to be placed around the skull and a network of at least one flexible band with voids therebetween, attached to the at least one first band and configured to ensure a link between the at least one first band and the at least one movable electrode module. According to one embodiment, the at least one movable electrode module comprises a lower part having a central opening and an upper part configured to couple together, at least one orifice, at least one electrical connecting means, and at least one aperture between said lower and upper parts, wherein at least one conductive cable extends through the orifice, the lower part is configured to receive at least one electrode through the central opening, the at least one electrical connecting means is configured to be connected to the at least one conductive cable and to the at least one electrode, and at least one flexible band extends through the at least one aperture so that the at least one movable electrode module can move along said flexible band. According to one embodiment, the at least one electrode is removable. According to one embodiment, the at least one electrode is a dry electrode. According to one embodiment, the at least one conductive cable is layered against the at least one flexible band, preferably the at least one conductive cable is layered against the at least one flexible band using at least one textile ring. According to one embodiment, the flexible bands network is configured to be articulated in the form of a network according to the measuring system 10/20. According to one embodiment, the electrode system is a headgear. According to one embodiment, the electrode system further comprises at least one electronic control unit comprising at least one of the following functional parts:

- an A/D interface;
- a signal processing part;
- a power supply;
- a wire communication part and/or a wireless communication part; and
- a micro-controller unit (MCU);

wherein said at least one control unit is connected to the at least one electrode by the at least one conductive cable. According to one embodiment, the electrode system further comprises at least two non-movable electrode modules positionable at different locations on the at least one flexible band, wherein the at least two non-movable electrode modules are fixed on said at least one flexible band, said at least two non-movable electrode modules define the network nodes of the flexible bands network, and the at least one movable electrode module is supported on at least one flexible band between two network nodes. According to one embodiment, the at least two non-movable electrode modules comprise a lower part having a central opening and an upper part configured to couple together, at least one attachment means on which at least one flexible band is attached, an orifice, and at least one electrical connecting means, wherein at least one conductive cable extends through the orifice, the lower part is configured to receive at least one electrode through the central opening, and the at least one electrical connecting means is configured to be connected to the at least one conductive cable and to the at least one electrode. According to one embodiment, the at least two non-movable electrode modules comprise at least one non-movable frontal electrode module configured to receive at least one frontal electrode and at least one non-movable cranial electrode module configured to receive at least one cranial electrode, wherein the at least one frontal electrode is configured to be in contact with the frontal skin of the subject when in the at least one frontal electrode module, and comprises a foam and conductive silver fibers; and the at least one cranial electrode is configured to be in contact with the skin of the subject through the hair when in the at least one cranial electrode module, and comprises a housing and a plurality of spring mounted pins. The present invention also relates to an electrode system comprising:

- at least one flexible band; and
- at least one electrode module comprising a lower part and an upper part configured to couple in a first position and in a second position;
- wherein the at least one flexible band passes between said lower and upper parts of at least one electrode module, and is retained in the first position or free to move in the second position.

**DEFINITIONS**

In the present invention, the following terms have the following meanings:

- "**Movable**" means having at least one degree of freedom.

- "**Non-movable**" means having no degree of freedom.

- "**Snap fastener**" refers to a pair of interlocking discs, made out of metal or plastic. A circular lip under one disc fits into a groove on the top of the other, holding them fast until a certain amount of force is applied. Snap fastener, snap button, press stud, popper, snap or tich are used interchangeably.

- "**Bio-signal**" refers to any signal in subjects that can be continually measured and monitored. Bio-signal refers especially to any biological parameter which can be measured by an instrument which converts a physical measure (light, pressure, electricity, radio-signal...) into an analogous signal (in volts) and which is then digitalized.

- "**Acquisition electrode**" refers to an active or passive electrode designed for measuring a bio-signal.

- "**Active electrode**" refers to an electrode comprising at least one amplifier and optionally other electronic components.

- "**10/20 system**" refers to an internationally recognized method describing the location of scalp electrodes in the context of an EEG test or experiment. The "10" and "20" refer to the fact that the actual distances between adjacent electrodes are either 10% or 20% of the total front-back or right-left distance of the skull. The letters F, T, C, P and O stand for frontal, temporal, central, parietal, and occipital lobes, respectively. Even numbers (e.g. 2, 4, 6, 8) refer to electrode positions on the right hemisphere, whereas odd numbers (e.g. 1,3,5, 7) refer to those on the left hemisphere. In addition, the letter codes A, Pg and Fp identifies the earlobes, nasopharyngeal and frontal polar sites respectively.

- "**10/10 system**" refers to an internationally recognized method describing the location of scalp electrodes in the context of an EEG test or experiment. The "10" refer to the fact that the actual distances between adjacent electrodes are 10% of the total front-back or right-left distance of the skull. The letters F, T, C, P and O stand for frontal, temporal, central, parietal, and occipital lobes, respectively. Even numbers (e.g. 2, 4, 6, 8) refer to electrode positions on the right hemisphere, whereas odd numbers (e.g. 1, 3, 5, 7) refer to those on the left hemisphere. In addition, the letter codes A, Pg and Fp identifies the earlobes, nasopharyngeal and frontal polar sites respectively.

- "**10/5 system**" refers to an internationally recognized method describing the location of scalp electrodes in the context of an EEG test or experiment. The "10" and "5" refer to the fact that the actual distances between adjacent electrodes are either 10% or 5% of the total front-back or right-left distance of the skull. The letters F, T, C, P and O stand for frontal, temporal, central, parietal, and occipital lobes, respectively. Even numbers (e.g. 2, 4, 6, 8) refer to electrode positions on the right hemisphere, whereas odd numbers (e.g. 1, 3, 5, 7) refer to those on the left hemisphere. In addition, the letter codes A, Pg and Fp identifies the earlobes, nasopharyngeal and frontal polar sites respectively.

- "**Contact**" refers to the immediate proximity with the skin of a subject providing a direct current path between an electrode and the subject's body for acquiring a bio-signal.

- "**Dry electrode**" refers to an electrode which does not necessitate the use of an electrolyte (for example a conductive gel) for acquiring a bio-signal, (e.g. an electrode comprising pins spring-loaded electrode).

- "**Ground electrode**" refers to an electrode configured to serve as a common reference point for all voltage in the system.

- "**Passive electrode**" refers to an electrode which does not comprise any amplifier.

- "**Pin**" refers to a rod-like or an elongated member comprising a first free end comprising a skin contact interface and a second end connected to an electronic interface.

- "**Reference electrode**" refers to an electrode to which signals received from another electrode can be compared as a potential difference in order to measure the voltage between the two electrodes.

- "**Subject**" refers to an animal, preferably a mammal, more preferably a human. The subject may be a patient, i.e. the subject is awaiting the receipt of, or is receiving medical care or is/will be the object of a medical procedure.

- "**Node**" refers to a point of intersection within a network.

## DETAILED DESCRIPTION

The following detailed description will be better understood when read in conjunction with the drawings. For the purpose of illustrating, the system is shown in the preferred embodiments. It should be understood, however that the application is not limited to the precise arrangements, structures, features, embodiments, and aspects shown. The drawings are not drawn to scale and are not intended to limit the scope of the claims to the embodiments depicted. Accordingly, it should be understood that where features mentioned in the appended claims are followed by reference signs, such signs are included solely for the purpose of enhancing the intelligibility of the claims and are in no way limiting on the scope of the claims.

*ELECTRODE SYSTEM*

This invention relates to an electrode system **1**.

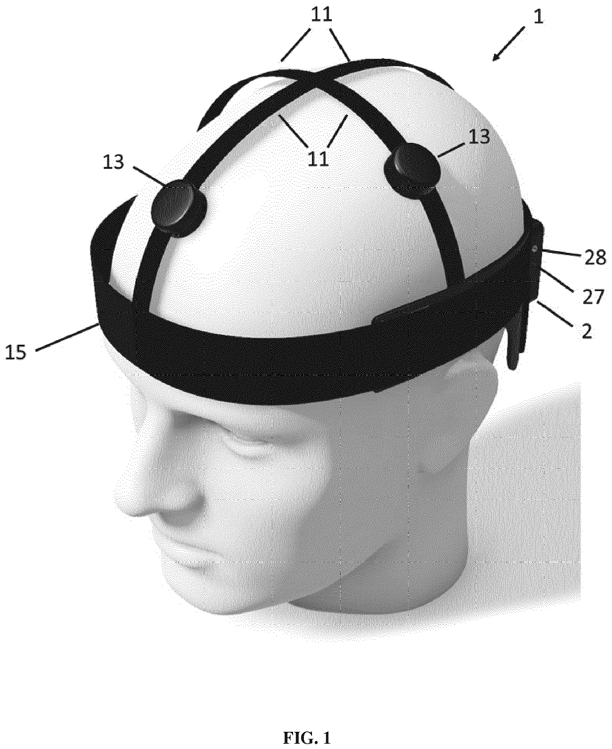

As illustrated on figure 1, the electrode system **1** comprises:

- at least one flexible band **11**;
- at least one movable electrode module **13** having at least one degree of freedom with reference to said at least one flexible band **11** and positionable at different locations on the at least one flexible band **11**;
- at least one conductive cable **14** connected to the at least one movable electrode module **13**; and
- at least one electrode configured to fit in one movable electrode module **13**, to be connected with the at least one conductive cable **14** and to detect at least one bio-signal.

According to one embodiment, the at least one flexible band **11** is a network of flexible bands.

The operator, or subject himself, places the electrode system **1** on the head of said subject. The flexible bands network will easily adjust to the head to adapt a snug fit. The at least one movable electrode module **13** allows the flexible bands **11** to be steadily held, and the pressure of the flexible bands network to be equally divided on each measuring points. Thus the at least one electrode has a good contact with the skin of the subject and allows for an enhanced signal to noise ratio. The electrode system **1** can be adapted to a wide range of electrode positions on the head of a subject by placing the at least one movable electrode module **13** at any wished electrode position. Then, the at least one electrode can be placed in one electrode module and the system is ready for acquisition in minimum time such as for example 30 seconds.

According to one embodiment, the electrode system **1** is configured to detect at least one bio-signal of a subject.

According to one embodiment, the electrode system **1** is configured to detect brain signals such as for example EEG signals of a subject.

According to one embodiment, the electrode system **1** is a headgear, a helmet or a hat.

According to one embodiment, the electrode system **1** is disposable. In this embodiment, the disposability ensures a good hygiene and saves time.

According to one embodiment, the electrode system **1** is designed for clinical or non-clinical application.

According to one embodiment, the electrode system **1** can be folded into a collapsed state for an easy storage. The at least one flexible band allows for a high flexibility of said electrode system **1**.

According to one embodiment, the electrode system **1** comprises a wireless transmitter/transceiver.

According to one embodiment, the pressure of the flexible bands network rages from 0.3 to 1.5 N. This embodiment ensures an impedance ranging from 1 to 200 kOhms.

According to one embodiment, the flexible bands network comprises at least one first band **15** configured to be placed around the skull, and a network of at least one flexible band **11**, with voids therebetween, attached to the at least one first band **15** and configured to ensure a link between the at least one first band **15** and the at least one movable electrode module **13**. In this embodiment, the flexible bands network leaves portions of the head uncovered, allowing the skin to breathe and enhancing the comfort of the subject.

According to one embodiment, the at least one first band **15** is flexible, semi-flexible or rigid.

According to one embodiment, the flexible bands network comprises at least one, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 flexible bands **11**.

According to one embodiment, the flexible bands network is configured to be articulated in the form of a network according to a measuring system 10/20, 10/10 or 10/5. In this embodiment, the at least one movable electrode module **13** can be positioned at different locations of the flexible bands network chosen in accordance with a desired electrode placement scheme (e.g. on parietal and occipital lobes) depending on nature of the measurement.

According to one embodiment, the at least one flexible band **11** has an elasticity configured to satisfy the following characteristics:

$$\frac{stretched\ length}{resting\ length} = 2.5$$

In this embodiment, the electrode system **1** is adjustable on multiple heads independently of the head size of the subject while keeping the right proportions between each measurement point.

According to one embodiment, the at least one flexible band **11** has an elasticity ranging from 2 to 3.5.

According to one embodiment, the at least one flexible band **11** is an elastic band, fabric band, nitrile band, or a mixture thereof.

According to one embodiment, the at least one flexible band **11** comprises a biocompatible material.

According to one embodiment, the at least one flexible band **11** is disposable. In this embodiment, the disposability ensures a good hygiene and saves time.

According to one embodiment, the at least one flexible band **11** is attached to the at least one first band **15** by sewing, gluing, or using a self-gripping band.

According to one embodiment, the at least one flexible band **11** has a predetermined length independently of the head size of the subject.

According to one embodiment, the at least one flexible band **11** has a width inferior to the width of the at least one first band **15**.

According to one embodiment, the at least one flexible band **11** has a width 1%, 2%, 3%, 4%, 5%, 10%, 15%, 20%, 25%, 30%, 35%, 40%, 45%, 50%, 55%, 60%, 65%, 70%, 75%, 80%, 85%; 90%, 95% smaller than the width of the first band **15**.

According to one embodiment, the at least one flexible band **11** has a width superior to the width of the at least one first band **15**.

According to one embodiment, the at least one flexible band **11** has a width equal to the width of the at least one first band **15**.

According to one embodiment, the at least one flexible band **11** has a width sufficient not to hurt or cut the skin of the subject.

According to one embodiment, the flexible bands **11** all have the same width.

According to one embodiment, the flexible bands **11** have different widths.

According to one embodiment, the electrode system **1** further comprises at least one hoop. In this embodiment, the at least one first band **15** is attached to the at least one hoop.

According to one embodiment, the at least one hoop is semi-flexible, or rigid.

According to one embodiment, the electrode system **1** further comprises at least one lateral rigid reinforcement attached to the at least one first band **15**.

According to one embodiment, the electrode system **1** comprises at least one, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30,31,32,33,34,35,36,37,38,39,40,41,42,43,44,45,46,47,48,49,50,51,52,53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, or 130 movable electrode modules **13**.

In a preferred embodiment, the electrode system **1** comprises 8, 16, 32, 64 or 128 movable electrode modules **13**.

According to one embodiment, the electrode system **1** comprises at least one empty electrode module. In this embodiment, at least one electrode module does not receive an electrode, said empty electrode module will enhance the contact and the secured positioning of the electrode system **1** on the head of the subject.

According to one embodiment, the at least one movable electrode module **13** is removably positioned on the at least one flexible band **11**.

According to one embodiment, the at least one movable electrode module **13** is disposable. In this embodiment, the disposability ensures a good hygiene and saves time.

According to one embodiment, the at least one movable electrode module **13** has a round, a rectangular, a triangular, a polygon shape, or any other shape.

According to one embodiment, the at least one movable electrode module **13** has a flat surface.

According to one embodiment, the at least one movable electrode module **13** has a curved surface.

According to one embodiment, the at least one movable electrode module **13** is disinfectable. In this embodiment, the at least one movable electrode module **13** is disinfected using a cleaning bath, a disinfectant wipe, or any other means known by one skilled in the art.

According to one embodiment, the at least one movable electrode module **13** comprises a lower part **131** and an upper part **132** configured to couple together.

According to one embodiment, the one movable electrode module **13** comprises a lower part **131** and an upper part **132** configured to couple together in a first position and in a second position, the at least one flexible band **11** passes between said lower **131** and upper **132** parts, and is retained in the first position or free to move in the second position.

According to one embodiment, the lower part **131** and the upper part **132** of the at least one movable electrode module **13** couple together by screwing, or by using at least one clip.

According to one embodiment, the lower part **131** and the upper part **132** of the at least one movable electrode module **13** comprise a plastic or an elastomer, such as for example polyamide, or acrylonitrile butadiene styrene.

According to one embodiment, the lower part **131** and the upper part **132** of the at least one movable electrode module **13** comprise a biocompatible material. In this embodiment, the at least one movable electrode module **13** is appropriate for a medical use, especially in a hospital environment.

According to one embodiment, the biocompatible material is certified to the requirements of USP Class VI.

According to one embodiment, the at least one movable electrode module **13** comprises means for receiving and fastening the at least one electrode.

According to one embodiment, the means for receiving and fastening the at least one electrode is a snap fastener, or a locking groove.

According to one embodiment, the means for receiving and fastening the at least one electrode is a female or a male snap fastener.

According to one embodiment, at least one extremity of the at least one conductive cable **14** is welded to the snap fastener. In this embodiment, the connection between the at least one conductive cable **14** and the at least one electrode is robust.

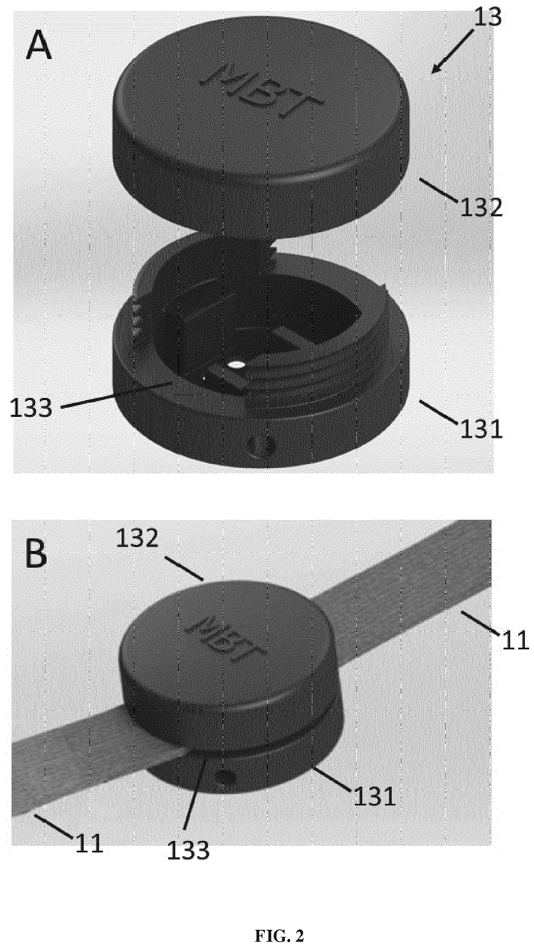

According to one embodiment, as illustrated on figure 2, the at least one movable electrode module **13** comprises a lower part **131** having a central opening and an upper part **132** configured to couple together, at least one orifice, at least one electrical connecting means, and at least one aperture **133** between said lower part **131** and upper part **132**, wherein at least one conductive cable **14** extends through the orifice, the lower part **131** is configured to receive at least one electrode through the central opening, the at least one electrical connecting means is configured to be connected to the at least one conductive cable **14** and to the at least one electrode, and at least one flexible band **11** extends through the at least one aperture **133** so that the at least one movable electrode module **13** can move along said flexible band **11** to be positioned at a chosen location. In this embodiment, the electrode system **1** is a system adjustable and adaptable to a multitude of measurement points according to a measuring system 10/20, 10/10, or 10/5, i.e. the operator has a wide choice of electrode positions on the head of a subject without having to change the electrode system **1**.

According to one embodiment, the at least one movable electrode module **13** is immobilized during the acquisition of signals by coupling the upper part **132** with the lower part **131** of said at least one movable electrode module **13**. In this embodiment, the electrode system **1** is a system adjustable and adaptable to a multitude of measurement points according to a measuring system 10/20, 10/10, or 10/5, i.e. the operator has a wide choice of electrode positions on the head of a subject without having to change the electrode system **1**.

According to one embodiment, at least one extremity of the at least one conductive cable **14** is welded to the at least one electrical connecting means. In this embodiment, the connection between the at least one conductive cable **14** and the at least one electrode is robust.

According to one embodiment, the at least one electrical connecting means comprises at least one PCB. In this embodiment, the at least one movable electrode module **13** carries the electronics specific to the at least one electrode.

According to one embodiment, the at least one PCB is configured to receive and process a raw signal from the at least one electrode and to provide an output signal.

According to one embodiment, the at least one PCB is located on the wall, in the lower part **131** or in the upper part **132** of the at least one movable electrode module **13**.

According to one embodiment, the at least one PCB comprises at least one connector to connect the at least one electrode.

According to one embodiment, the at least one movable electrode module **13** is configured to receive at least one cranial electrode **17**. According to one embodiment, the at least one movable electrode module **13** is configured to receive at least one frontal electrode **16**.

According to one embodiment, the at least one movable electrode module **13** has a height ranging from 1 to 50 mm.

According to one embodiment, the height of the at least one movable electrode module **13** is preferably 8 mm.

According to one embodiment, the at least one movable electrode module **13** has a diameter ranging from 1 to 50 mm.

According to one embodiment, the diameter of the at least one movable electrode module **13** is preferably 15 mm.

According to one embodiment, the electrode system **1** comprises at least one, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30,31,32,33,34,35,36,37,38,39,40,41,42,43,44,45,46,47,48,49,50,51,52,53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, or 130 electrodes.

In a preferred embodiment, the electrode system **1** comprises 8, 16, 32, 64, or 128 electrodes.

According to one embodiment, the at least one electrode is a dry electrode. In this embodiment, the at least one electrode doesn't require any gel. This embodiment permit a quick preparation of the at least one electrode. Moreover, the use of dry electrodes allows a good conduction between the electrodes and the skin of the subject.

According to one embodiment, the at least one electrode is removable. In this embodiment, the at least one electrode is independent of the at least one flexible band **11** and the at least one movable electrode module **13**. Furthermore, a removable electrode allows for an easy and quick installation and removal of said electrode in the at least one movable electrode module **13**.

According to one embodiment, the at least one electrode is waterproof. In this embodiment, the at least one electrode is disinfectable. In this embodiment, the at least one electrode is disinfected using a cleaning bath, a disinfectant wipe, or any other means known by one skilled in the art.

According to one embodiment, the at least one electrode is not waterproof. In this embodiment, the at least one electrode is disposable.

According to one embodiment, the at least one electrode is disposable. In this embodiment, the disposability ensures a good hygiene and saves time. In this embodiment, the at least one electrode is changed after every use.

According to one embodiment, the at least one movable electrode module **13** and the at least one electrode are disposable. In this embodiment, the disposability ensures a good hygiene and saves time.

According to one embodiment, the at least one electrode comprises a lower part and an upper part configured to couple together.

According to one embodiment, the lower part and the upper part of the at least one electrode couple together with a pivot connection, by screwing, or by using at least one clip.

According to one embodiment, the upper part of the at least one electrode comprises at least one centering strip compatible with the lower part of said at least one electrode configured to secure the coupling of the upper part with the lower part.

According to one embodiment, the at least one electrode comprises at least one tension spring.

According to one embodiment, the at least one electrode comprises means to be fastened in one electrode module.

According to one embodiment, the means to be fastened in one electrode module is a snap fastener or at least one projection.

According to one embodiment, the at least one electrode comprises a female or a male snap fastener.

According to one embodiment, the at least one electrode comprises a female snap fastener and the at least one movable electrode module **13** comprises a male snap fastener. In this embodiment, the at least one electrode is easily fastened in the at least one movable electrode module **13**, and the overload of the electrode system **1** is kept to a minimum.

According to one embodiment, the at least one electrode comprises a male snap fastener and the at least one movable electrode module comprises a female snap fastener. In this embodiment, the at least one electrode is easily fastened in the at least one movable electrode module **13**, and the overload of the electrode system **1** is kept to a minimum.

According to one embodiment, the at least one electrode comprises at least one means to be connected to the at least one electrical connecting means of one electrode module **166**.

According to one embodiment, the at least one means to be connected to the at least one electrical connecting means of one electrode module is the same as the means to be fastened in one electrode module.

According to one embodiment, the at least one means to be connected to the at least one electrical connecting means of one electrode module is a snap fastener or a cable or a wire or any other connecting means.

According to one embodiment, the at least one movable electrode module **13** comprises at least one locking groove and the upper part of the at least one electrode comprises at least one projection configured to fit in said locking groove to fasten the at least one electrode in the at least one movable electrode module **13**.

According to one embodiment, the lower part of the at least one electrode comprises at least one recess and the at least one movable electrode module **13** comprises at least one projection configured to fit in said recess. In this embodiment, coupling of the projection in the recess ensure alignment of the connectors of the at least one electrode and the at least one movable electrode module **13**.

According to one embodiment, the electrode system **1** comprises a ground electrode, a reference electrode and/or at least one acquisition electrode.

Bio-signal records are bipolar i.e. they represent the difference in potential between the acquisition electrode of interest, and a reference electrode. In one embodiment, the reference and ground electrodes are placed on the skin to measure brain signal compared to another brain signal as reference. Biological activity (such as brain activity) but also environmental electric and magnetic fields may generate skin difference in potential. Therefore the electrode system **1** of the invention also comprises a ground electrode which isolates a subject from the ground of the power supply. This configuration comprising a ground, a reference and an acquisition electrode is designed to reject the spatially constant common-mode potential and amplify the difference in potential between pairs of skin locations such that the output voltage is proportional to skin difference in potential generated within the body. Impedances for all acquisition electrodes are compared to both the ground and the reference electrode during data processing.

According to one embodiment, the electrode system **1** comprises at least one ground electrode and at least one reference electrode. In some embodiment, both reference and ground electrodes are configured to be located behind the ears, on the mastoids. According to one embodiment, acquisition electrodes are placed on area where skin voltage is changing and reference electrode on a neutral site i.e. an area where the skin voltage varies as little as possible. According to one embodiment, the reference electrode is placed on the mastoids, or vertex. According to another embodiment, the reference is calculated by averaging the signal of several acquisition electrodes. According to one embodiment, the reference electrode is located on the right side mastoid. According to one embodiment, the reference electrode is located on the left side mastoid. Mastoids are two ideal locations to measures non-brain potentials with a minimal amount of artifacts.

According to one embodiment, the ground electrode is placed on the forehead of the subject. In some embodiment, reference electrode is located at any location on a subject head. According to one embodiment, the ground electrode is placed on ear location of the subject. According to one embodiment, said ground electrode is located on left side mastoid. According to one embodiment, said ground electrode is located on right side mastoid or on the frontal area.

According to one embodiment, the electrode system **1** comprises two electrodes respectively located on the right and on the left hemisphere; and at least one electrode placed on the parietal lobe. In one embodiment, the electrode system **1** comprises two electrodes respectively located on the right and on the left hemisphere. According to one embodiment, the electrode system **1** comprises several acquisition electrodes, for example 1, 4, 5, 8, 10, 16, 20, 25, 30, 40, or 50 acquisition electrodes. In one embodiment, the electrode system **1** may comprise an equal number of acquisition and reference electrodes, an equal number of acquisition and ground electrodes and/or an equal number of both acquisition, ground and reference electrodes.

According to one embodiment, the at least one electrode is an active electrode. According to one embodiment, the at least one electrode is a passive electrode. According to one embodiment, the ground and/or the reference electrodes are passive electrodes. According to one embodiment, all the acquisition electrodes are passive electrodes. According to one embodiment, reference and ground electrodes are active electrodes.

According to one embodiment, the active electrode comprises at least one amplifier, said amplifier having a gain ranging from 1 to 5000, from 1 to 2500, from 1 to 1000, or from 1 to 500. According to one embodiment, the active electrode comprises an impedance converter. According to one embodiment, the active electrode comprises an amplifier and set of protections such as Transient Voltage Suppression (TVS) diodes and signal frequency filters for example. According to one embodiment, the active electrode comprises an amplifier, a set of protections such as Transient Voltage Suppression (TVS) diodes and signal frequency filters for example and a set of electrical protections for the user.

According to one embodiment, the active electrode comprises an amplifier which has a low intrinsic noise (<76 nV P-P) in the frequency range of 0.1 to 10 Hz for example. The amplifier may have a low drift and low offset voltage. This configuration enables to provide the best separation of bio-signal from interference signals and noise. In some embodiment, the amplifier has a high common mode rejection ratio for example at least 110 dB.

According to one embodiment, the at least one electrode is electrically connected to at least one PCB located in one electrode module **13** configured to receive and process a raw signal from the at least one electrode and to provide an output signal.

Advantageously, the ground electrode enables to improve and optimize noise reduction in the amplifier, particularly common mode rejection. A ground electrode is needed to serve as a common reference point for all voltage in the system.

According to one embodiment, the at least one frontal electrode **16** is configured to be in contact with the frontal skin of the subject when in one electrode module.

According to one embodiment, the at least one frontal electrode **16** is a textile electrode. In this embodiment, the textile electrode ensures comfort for the subject.

According to one embodiment, the at least one frontal electrode **16** comprises a foam and conductive fibers, such as for example conductive silver fibers.

According to one embodiment, the at least one frontal electrode **16** comprises silver-plated fibers integrated in a foam.

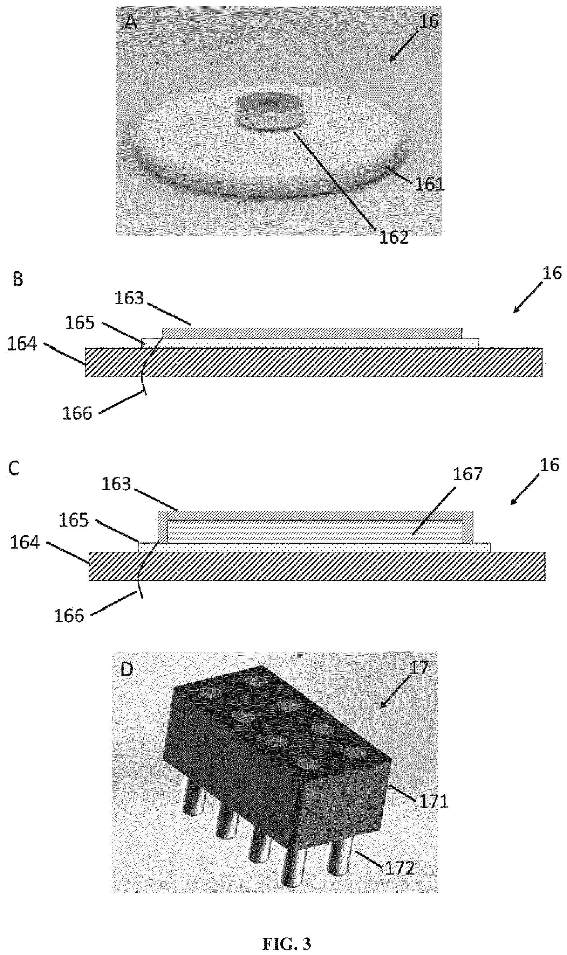

According to one embodiment, as illustrated on figure 3A, the at least one frontal electrode **16** comprises a foam coated with a conductive fabric **161**, such as for example a conductive silver fabric.

According to one embodiment, as illustrated on figure 3A, the at least one frontal electrode **16** comprises a foam coated with a conductive fabric **161** and a snap fastener **162**.

According to one embodiment, as depicted in figure 3B, the textile electrode comprises a conductive fabric **163** for application to the skin of a user, a support **164** and a relatively impermeable layer **165** in-between.

According to the applicant, the relatively impermeable layer, which exhibits low breathability, ensures retention of the perspiration of the subject between the skin and the relatively impermeable layer. The water or moisture secreted by the subject retains by the relatively impermeable layer lowers the contact impedance between the skin and the conductive fabric and therefore increases the signal-to-noise ratio.

As depicted on table 1, the use of such relatively impermeable layer lowers the contact impedance, especially when the subject wear the textile electrode for at least a few minutes. After the early stages of wearing the textile electrodes, perspiration is trapped between the skin and the impermeable layer, thereby lowering the contact impedance between the skin and the conductive fabric.

Table 1

| Time after application of the textile electrode on the skin of the subject (minutes) | 0mn | 3mn | 9mn | 16mn |
|---|---|---|---|---|
| Contact impedance with impermeable layer according to the invention (kOhms) | 165 | 116 | 89 | 79 |
| Contact impedance for standard electrode (without impermeable layer) (kOhms) | 165 | 145 | 133 | 134 |

According to one embodiment, the conductive fabric is configured to be applied on the forehead of a subject or behind the ear at the level of the mastoid process.

According to one embodiment, the conductive fabric is knitted or woven with conductive yarns, such as silver yarns. According to an alternative embodiment, the conductive fabric is knitted or woven with non-conductive yarns and further comprise embedded conductive yarns.

According to one embodiment, the support is a foam or a textile layer.

According to one embodiment, the relatively impermeable layer is a relatively moisture impermeable layer. According to one embodiment, the relatively impermeable layer is a polymeric layer.

According to one embodiment, the water vapor transmission rate of the relatively impermeable layer is lower than 1000 g/m$^2$/day, 500 g/m$^2$/day, 100 g/m$^2$/day, 50 g/m$^2$/day, 25 g/m$^2$/day or 10 g/m$^2$/day.

According to one embodiment, as depicted on figures 3B and 3C, the area of the relatively impermeable layer is equal or higher than the area of the conductive fabric in order to ensure retention of perspiration and moisture over the entire area of the conductive fabric.

According to another embodiment, the area of the relatively impermeable layer is lower than the area of the conductive fabric in order to ensure retention of perspiration and moisture on specific area of the conductive fabric.

According to one embodiment, as depicted in figure 3C, the textile electrode further comprises an absorbent material **167** between the relatively impermeable layer and the conductive fabric.

According to the Applicant, positioning an absorbent material between the conductive fabric and the relatively impermeable layer ensures storage of water or moisture. Said absorbent material ensures moisturizing the conductive fabric over time.

According to one embodiment, the absorbent material is made of an hygroscopic material. According to one embodiment, the absorbent material is a foam or a sponge-like material.

According to one embodiment, the foam is a low-density foam such as for example urethane foam.

According to one embodiment, the at least one frontal electrode **16** is a passive electrode. According to one embodiment, the at least one frontal electrode **16** is an active electrode. According to one embodiment, the at least one frontal electrode **16** is a ground electrode. According to one embodiment, the at least one frontal electrode **16** is a reference electrode. According to one embodiment, the at least one frontal electrode **16** is an acquisition electrode.

According to one embodiment, the at least one cranial electrode **17** is configured to be in contact with the skin of the subject through the hair when in the at least one cranial electrode module.

According to one embodiment, the at least one cranial electrode **17** is a passive electrode. According to one embodiment, the at least one cranial electrode **17** is an active electrode. According to one embodiment, the at least one cranial electrode **17** is a ground electrode. According to one embodiment, the at least one cranial electrode **17** is a reference electrode. According to one embodiment, the at least one cranial electrode **17** is an acquisition electrode.

According to one embodiment, the at least one cranial electrode **17** is a spring-loaded electrode.

According to one embodiment as illustrated on figure 3D, the at least one cranial electrode **17** comprises a housing **171** and at least one pin **172**.

According to one embodiment as illustrated on figure 3D, the at least one cranial electrode **17** comprises at least one pin **172**.

According to one embodiment, the at least one pin **172** has a first free end comprising a skin contact interface and a second end connected to an electronic interface.

According to one embodiment, the first free end is configured to be soft or smooth to improve the comfort of the electrode system **1**.

According to one embodiment, the second end is connected to a surface mounted device and forms an electronic interface allowing an electronic connection between the at least one pin **172** and a PCB for processing the bio-signal.

According to one embodiment, the at least one pin **172** is configured for passing across the hairs while ensuring a conductive contact.

According to one embodiment, the at least one pin **172** is a rod-like member having a diameter ranging from 0.25 mm to 1 cm, or from 1 to 2 mm.

According to one other embodiment, the length of the at least one pin **172** can be variable and length is ranging from 1 mm to 20 mm, or 4.5 to 7.5 mm. Advantageously, the variability of the length enables to choose the optimal length depending on the head location, the hair length and density and more generally, depending on the subject morphology.

In one embodiment, dimensions of the at least one pin **172** and material of the at least one pin **172** are configured to provide contact impedance ranging from $1\Omega$ to 300 k$\Omega$. This impedance will decrease with the number of pins **172** and their electrically parallel connection, regardless of the nature of the electrode (i.e. active or passive).

According to one embodiment, the at least one pin **172** is made in a conductive material forming an elongated protrusion. According to one embodiment, said elongated protrusion has any shape providing sufficient and painless contact with the subject skin through the hair, for example a cylindrical, triangular or rectangular shape with a rounded free end. According to one embodiment, the at least one pin **172** is coated with a conductive material such as for example gold. According to one embodiment, the at least one pin **172** comprises a material which is waterproof, durable and biocompatible for skin contact.

According to one embodiment, the at least one pin **172** imposes a pressure applied by said pin **172** in contact with the skin which is less than 5 N, for example 0.25 N, 0.75 N, 1 N, 2 N, 3 N, 4 N, 5 N or less.

According to one embodiment, the at least one cranial electrode **17** comprises at least one row of 1, 2, 3, 4, 5, 6, 7, 8, 9, 10, 11, 12, 13, 14, 15, 16, 17, 18, 19, or 20 pins **172**.

According to one embodiment, the at least one cranial electrode **17** preferably comprises two rows of 4 pins **172**.

According to one embodiment, the rows of at least one pin **172** are arranged electrically in parallel. In this embodiment, the electrically parallel configuration enables to decrease the contact impedance while acquiring easily much more head points across the hairs. The use of active electrodes also contributes to reduce the contact impedance.

According to one embodiment, the distance between two pins **172** is ranging from 0.25 mm to 10 mm, or 2 mm to 3 mm.

According to one embodiment, the central opening of the at least one movable electrode module **13** comprises a number of through holes equal to the number of pins **172**. In this embodiment, each pin **172** of the at least one cranial electrode **17** exits the at least one movable electrode module **13** by one of the said through holes.

According to one embodiment, the at least one cranial electrode **17** comprises a case configured to receive the housing **171** and the at least one pin **172**. In this embodiment, the case comprises the means to be fastened in one electrode module.

According to one embodiment, the case comprises two projections to facilitate the removal of the at least one electrode from one electrode module.

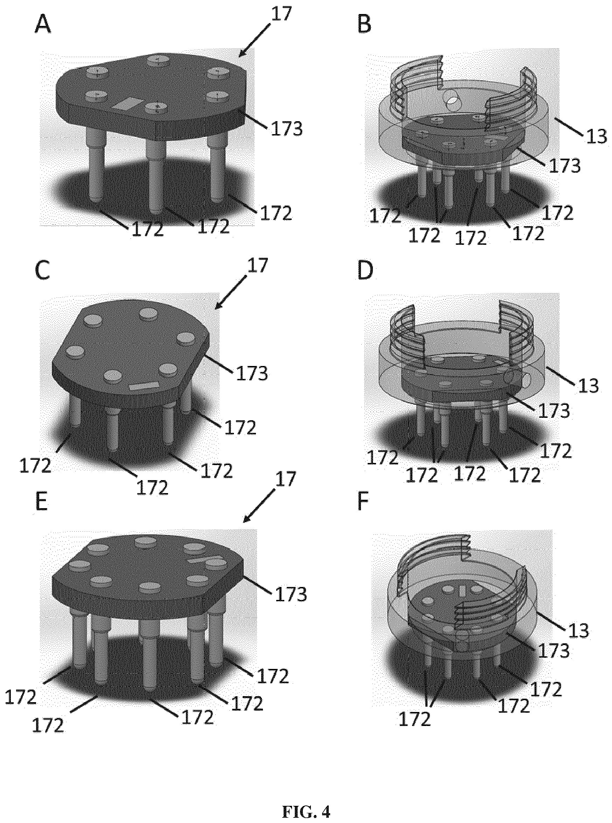

According to one embodiment, illustrated on figure 4, the at least one cranial electrode **17** comprises a PCB **173**, wherein the at least one pin **172** is integrated in said PCB **173**. In this embodiment, the at least one cranial electrode is ready to be connected to the at least one electrical connecting means of one electrode module **13**.

According to one embodiment, said PCB **173** is a chamfered disk configured to fit in the lower part **131** of one electrode module **13**.

According to one embodiment, illustrated on figures 4A-4F, said PCB **173** has a round, a rectangular, a triangular, a polygon shape, or any other shape.

According to one embodiment, the at least one conductive cable **14** passes through the orifice **124** of the at least one movable electrode module **13** in order to go through said at least one movable electrode module **13** to reach the next electrode module **13**.

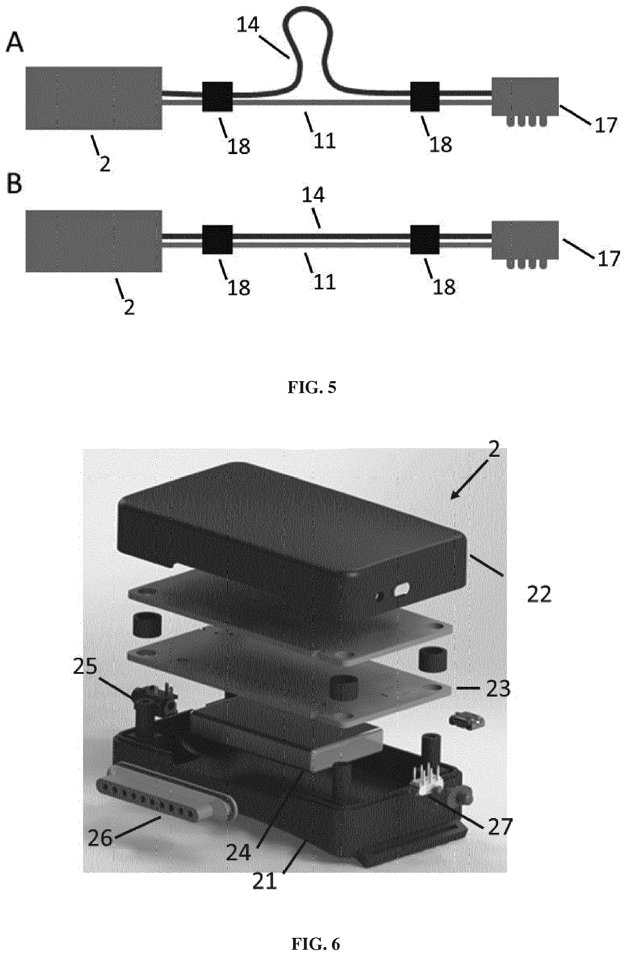

According to one embodiment, as illustrated on figures 5A and 5B, the at least one conductive cable **14** is layered against the at least one flexible band **11**, preferably the at least one conductive cable **14** is layered against the at least one flexible band **11** using at least one textile ring **18**. In this embodiment, the electrode system **1** stays compact with limited disorder due to conductive cables **14**, thus preventing any discomfort or difficulties in the case of a subject already heavily medically equipped. Moreover, in the case of a subject who is not in control of his movements, the risk of involuntary removal of the at least one conductive cable **14** is prevented.

According to one embodiment, the at least one conductive cable **14** is in a sliding connection with the at least one flexible band **11** on which it is layered.

According to one embodiment, as illustrated on figure 5A, the at least one conductive cable **14** is connected to at least one movable electrode module **13** and layered against the flexible band **11** supporting said at least one movable electrode module **13**. In this embodiment, the length of the at least one conductive cable **14** is equal to the maximal stretched length of said flexible band **11** (figure 5B).

According to one embodiment, the at least one conductive cable **14** is configured to transport an EEG signals from the electrodes.

According to one embodiment, the at least one conductive cable **14** is a shielded cable.

According to one embodiment, at least one extremity of the at least one conductive cable **14** is welded to at least one movable electrode module **13**. In this embodiment, the at least one conductive cable **14** is connected to the at least one electrode upon reception of said at least one electrode in the corresponding movable electrode module **13**.

According to one embodiment, at least one extremity of the at least one conductive cable **14** is welded to the at least one electrical connecting means of at least one movable electrode module **13**.

According to one embodiment, the at least one conductive cable **14** is an electrical cable, a conductive plastic cable, or a mixture thereof.

According to one embodiment, the electrode system **1** comprises at least one, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30,31,32,33,34,35,36,37,38,39,40,41,42,43,44,45,46,47,48,49,50,51,52,53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 conductive cables **14**. In this embodiment, the conductive cables **14** form a network of conductive cables.

According to one embodiment, the network of conductive cables is layered against the at least one flexible band **11**, preferably the network of conductive cables is layered against the at least one flexible band **11** using at least one textile ring **18**.

According to one embodiment, the electrode system **1** further comprises at least one fitting means to attach the at least one flexible band **11** about the head.

According to one embodiment, the at least one fitting means is attached to the at least one first band **15**.

According to one embodiment, the two extremities of the at least one fitting means are attached to the at least one first band **15**.

According to one embodiment, the at least one fitting means is attached to the at least one hoop.

According to one embodiment, the two extremities of the at least one fitting means are attached to the at least one hoop.

According to one embodiment, the at least one fitting means comprises a chin strap, a front strap and/or an ear strap.

According to one embodiment, the at least one fitting means comprises at least one, 1, 2, 3, 4, 5 bands.

According to one embodiment, the chin strap divides into two bands converging in one band below the ears.

According to one embodiment, the at least one fitting means comprises means for attachment.

According to one embodiment, the means for attachment of the at least one fitting means is a clamping clip, or a self-gripping band. In this embodiment, the electrode system **1** is easily and quickly positioned and removed.

According to one embodiment, the at least one fitting means comprises at least one tightening loop configured to allow a quick adjustment and tightening of the electrode system **1**. In this embodiment, the adjustment of the electrode system **1** is easy, fast and precise.

According to one embodiment, the at least one tightening loop is located below at least one ear.

According to one embodiment, the at least one fitting means is disinfectable. In this embodiment, the at least one fitting means is disinfected using a cleaning bath, a disinfectant wipe, or any other means known by one skilled in the art.

According to one embodiment, as illustrated on figure 6, the electrode system **1** further comprises at least one electronic control unit **2** comprising at least one of the following functional parts:

- an A/D interface;
- a signal processing part;
- a power supply;
- a wire communication part and/or a wireless communication part; and
- a micro-controller unit (MCU) **23**;

wherein said at least one control unit **2** is connected to the at least one electrode by the at least one conductive cable **14**.

According to one embodiment, the at least one electronic control unit **2** comprises a lower part **21** and an upper part **22** configured to couple together.

According to one embodiment, the A/D interface is designed for digitizing the analogical signal. According to one embodiment, the A/D interface comprises a signal input multiplexer and/or comprises a programmable gain amplifier and/or comprises means for impedance measurement. For example, the A/D interface comprises an input signal amplifier, a programmable gain amplifier, an analog to digital converter and/or an impedance measurement.

According to one embodiment, the at least one electronic control unit **2** comprises an Analogical to Digital (A/D) converter.

According to one embodiment, said A/D converter after amplification coded with a resolution of 24 bits has a Signal to Noise Ratio (SNR) of 120 dB per channel.

According to one embodiment, the signal processing part is configured for analyzing the signal. An exemplary pre-processing algorithm comprises measuring the quality of the acquisition, imposing digital filter and/or encoding/crypting data.

According to one embodiment, the wire or the wireless communication parts are designed for transmitting the signal to an external device, for example a smartphone, a computer, or a tablet computer.

According to one embodiment, a MCU **23** is designed for controlling all or a part of the functional parts connected to the MCU **23**.

According to one embodiment, said MCU **23** is connected for sending and receiving data from means for pre-processing an algorithm and wireless communication means.

According to one embodiment, said MCU **23** is a PCB.

According to one embodiment, the at least one electronic control unit **2** comprises a signal amplifier. The signal needs to be amplified to make it compatible with devices such as displays, recorders or A/D converters.

According to one embodiment, the amplifier is selected in order to provide amplification selective to the bio-signal, and reject superimposed noises for both subjects and electronic components of the at least one electrode.

According to one embodiment, the at least one electronic control unit **2** comprises an impedance converter comprising an amplifier.

According to one embodiment, the at least one control unit **2** comprises a low-pass signal filter. According to one embodiment, the low-pass filter is in the order 1 to 8. Advantageously, the low-pass filter is configured to protect bio-signals from non-bio-signals such as environmental interferences.

According to one embodiment, the amplification is based on impedance conversion (i.e. to pass from a high to low impedance) using an amplifier, preferably an ultra-low noise amplifier, of gain equal to 1.

According to one other embodiment, the amplification is based on signal amplification with higher gain values with a configuration enabling a gain at least equal 2, for example a gain of 10, 20, 30, 40, 50, 100 for classical programmable gain amplifier or 1000, 5000 or 106 20 in the case of high gain amplifier. Advantageously, the amplification allows the quality of the bio-signal acquisition to be improved and readable.

According to one embodiment, the at least one electronic control unit **2** comprises a highpass filter for reducing low frequencies coming from bioelectric flowing potentials (e.g. breathing, cardiac activity...), for example with a cut-off frequency ranging from 0,1 to 2 Hz, or 0,1 to 0,7 Hz, or equal to 0,4 Hz.

Advantageously, the at least one electronic control unit **2** amplifies the bio-signal to a treatable level for a more precise and easier measurement of the signal, especially EEG wherein the level of signal is excessively fine (i.e. several tens of microvolts).

According to one embodiment, the signal processing part is a printed circuit board.

According to one embodiment, the power supply is a battery **24** or a power supply wire.

According to one embodiment, the battery **24** is a lithium ion battery.

According to one embodiment, the battery **24** has an autonomy 1h, 2h, 3h, 4h, 5h, 6h, 7h, 8h, 9h, 10h, 11h, 12h, 24h, 48h, 72h, or 96h.

According to one embodiment, the battery **24** is glued or screwed to the lower part **21** of the at least one electronic control unit **2**.

According to one embodiment, the at least one electronic control unit **2** comprises a socket **25** to plug a power supply wire.

According to one embodiment, the socket **25** to plug a power supply wire is a jack socket, preferably a mini jack socket.

According to one embodiment, the at least one electronic control unit **2** comprises a battery **24** and a socket **25** to plug a power supply wire. In this embodiment, the at least one electronic control unit **2** is powered using a battery **24**, and the power supply wire is used for loading said battery **24**.

According to one embodiment, the at least one electronic control unit **2** comprises a wire communication link, preferably an usb port or any other socket **25** configured to receive a means for loading the battery **24** of said at least one electronic control unit **2**.

According to one embodiment, the wire communication link is also suitable for installing a firmware, or exporting stored data.

According to one embodiment, the at least one electronic control unit **2** comprises an orifice to receive the at least one conductive cable **14**.

According to one embodiment, the at least one conductive cable **14** is welded to the MCU **23**.

According to one embodiment, the at least one electronic control unit **2** comprises at least one connector **26** welded to the signal processing part.

According to one embodiment, the at least one conductive cable **14** is connected to the at least one connector **26**, thus to the at least one electronic control unit **2**.

According to one embodiment, the at least one connector **26** is a device allowing the connection of a plurality of conductive cables **14** to the at least one electronic control unit **2**.

According to one embodiment, the at least one electronic control unit **2** and the at least one conductive cable **14** are connected via a male/female connection.

According to one embodiment, the at least one electronic control unit **2** and the at least one conductive cable **14** are removably connected.

According to one embodiment, the at least one electronic control unit **2** comprises means for switching on said at least one electronic control unit **27**.

According to one embodiment, the means for switching on the at least one electronic control unit **27** is a button or a switch, preferably a O/I switch.

According to one embodiment, the at least one electronic control unit **2** comprises means for indicating the state of connection and/or the state of ignition and/or condition of the battery **24** of the at least one electronic control unit **2**.

According to one embodiment, means for indicating the state of connection and/or the state of ignition and/or condition of the battery **24** of the at least one electronic control unit **2** is at least one light emitting diode (LED) **28**.

According to one embodiment, the at least one LED **28** is a blue, green, red LED, or a mixture thereof.

According to one embodiment, the at least one LED **28** is coupled with the means for switching on the at least one electronic control unit **27** so that the at least one LED **28** emits light and/or blink when said at least one electronic control unit **2** is switched on.

When the at least one electronic control unit **2** is switched on, the at least one LED **28** blinks blue light until the connection with the electrode system **1** is assured; then the at least one LED **28** emits continuously blue light; when the data acquisition is started, the light turns green; when the at least one electronic control unit **2** is charging on the mains, the light turns red. The installation of the electrode system **1** is easy and fast, requiring little action from the operator or the subject.

According to one embodiment, the at least one electronic control unit **2** comprises at least one socket to receive at least one means for connecting at least two electronic control units **2** together. In this embodiment, the synchronization of two electrode systems **1** is done by said at least one socket which provides isolated Transistor-Transistor Logic signal input to retrieve an external synchronization signal, this signal is temporally superimposed on the measured EEG data to synchronize in time with this external stimulation. To synchronize two electrode systems **1**, the same synchronized signal is sent to said at least one socket inputs of these two electrode systems **1** simultaneously. Once the data have been retrieved, it is then possible to synchronize them temporally with this synchronization signal. In this embodiment, at least two electrode systems **1** each comprising its own electronic control unit **2** can be coupled. In this embodiment, the at least one LED **28** configured to emit light and/or blink when the connection between said at least two electrode systems **1** is assured. In this embodiment, it is possible to observe correlations between different subjects, since the acquired signals are synchronized temporally.

According to one embodiment, the at least one socket to receive at least one means for connecting at least two electronic control units **2** together is a jack socket, preferably a mini jack socket, preferably a 3.5 mm mini jack socket.

According to one embodiment, the at least one electronic control unit **2** comprises means for controlling the recording of signals from the at least one movable electrode module **13**.

According to one embodiment, the means for controlling the recording of signals from the at least one movable electrode module **13** is a button or a switch, preferably a O/I switch.

According to one embodiment, the means for controlling the recording of signals from the at least one movable electrode modules **13** is of different color compared to the electronic control unit **2**.

According to one embodiment, the means for switching on the at least one electronic control unit **27** and the means for controlling the recording of signals from the at least one movable electrode module **13** are one and the same.

According to one embodiment, the at least one electronic control unit **2** is located on the at least one flexible band **11**. According to one embodiment, the at least one electronic control unit **2** is not located on the at least one flexible band **11**.

According to one embodiment, the at least one electronic control unit **2** is attached to the at least one first band **15**.

According to one embodiment, the at least one electronic control unit **2** comprises at least one handle, notch, slit, orifice or hole configured to receive at least one flexible or rigid band for attachment to the at least one flexible band **11**, or to the at least one first band **15**. In this embodiment, the flexibility of the electrode system **1** is preserved and no discomfort is caused to the subject.

According to one embodiment, the at least one electronic control unit **2** is located on the side, the front, or at the back of the head of the subject.

According to one embodiment, when located on the head of the subject, the lower part **21** of the at least one electronic control unit **2** is curved and comprises a layer of foam to ensure optimum comfort. In this embodiment, the lower part **21** is in contact with the head of the subject, the curvature of said lower part **21** and the layer of foam ensure a good position of the at least one electronic control unit **2** on the head of the subject while providing comfort to said subject.

According to one embodiment, the at least one electronic control unit **2** is positioned at a location of the electrode system **1** that allow the subject to be lying down comfortably. In this embodiment, the at least one electronic control unit **2** is not positioned at the back of the head of the subject.

According to one embodiment, the at least one electronic control unit **2** is placed on a table or a bed, preferably the at least one electronic control unit **2** is clipped on the side of a table, a bed, or any piece of furniture.

According to one embodiment, the lower part **21** and the upper part **22** of the at least one electronic control unit **2** comprise a plastic or an elastomer, such as for example polyamide, or acrylonitrile butadiene styrene.

According to one embodiment, the lower part **21** and the upper part **22** of the at least one electronic control unit **2** comprise a biocompatible material. In this embodiment, the at least one electronic control unit **2** is appropriate for a medical use, especially in a hospital environment.

According to one embodiment, the biocompatible material is certified to the requirements of USP Class VI.

According to one embodiment, the lower part **21** and the upper part **22** of said at least one electronic control unit **2** couple together by using at least one screw, glue, at least one clip, or any other fixation means.

According to one embodiment, the at least one electronic control unit **2** is waterproof.

According to one embodiment, the at least one electronic control unit **2** comprises a seal or an O-ring between the lower part **21** and the upper part **22** to ensure waterproofness of said at least one electronic control unit **2**.

According to one embodiment, the at least one electronic control unit **2** has a length ranging from 5 to 300 mm.

According to one embodiment, the at least one electronic control unit **2** has a width ranging from 5 to 300 mm.

According to one embodiment, the at least one electronic control unit **2** has a height ranging from 5 to 100 mm.

According to one embodiment, the at least one electronic control unit **2** has a weight ranging from 1 to 500 g.

According to one embodiment, the at least one electronic control unit **2** is disposable.

According to one embodiment, the at least one electronic control unit **2** is disinfectable. In this embodiment, the at least one electronic control unit **2** is disinfected using a disinfectant wipe, or any other means known by one skilled in the art.

According to one embodiment, the electrode system **1** comprises at least one wire guide system to guide the at least one conductive cable **14** from the at least one flexible band **11** to said at least one electronic control unit **2**. In this embodiment, the at least one conductive cable **14** exiting of the at least one electronic control unit **2** is protected by the at least one wire guide system, thus increasing the longevity of said at least one conductive cable **14**. In case of a plurality of conductive cables **14**, this embodiment prevents their interlacing. Thus, the electrode system **1** can be used intensively without damaging the at least one conductive cable **14** or the connection between said at least one conductive cable **14** and the at least one electronic control unit **2**.

According to one embodiment, the at least one wire guide system is disinfectable. In this embodiment, the at least one wire guide system is disinfected using a cleaning bath, a disinfectant wipe, or any other means known by one skilled in the art.

According to one embodiment, the at least one wire guide system is a textile sheath, a plastic sheath, or a braided sheath.

According to one embodiment, the at least one wire guide system has a length ranging from 1 to 500 cm.

According to one embodiment, the at least one wire guide system has preferably a length of 100 cm. In this embodiment, the subject is free of his movements, while the risks of hooking in the various objects present in his environment are minimized.

According to one embodiment, the at least one wire guide system is located at the back, at the side or at the front of the electrode system **1**.

According to one embodiment, the at least one wire guide system comprises at least one connector compatible with the at least one connector **26** of the at least one electronic control unit **2**. In this embodiment, the at least one wire guide system is connected to the at least one connector **26** of the at least one electronic control unit **2**, thus to the at least one electronic control unit **2**. In this embodiment, the operator and/or the subject does not have to connect all the conductive cables **14**, one by one, to the at least one electronic control unit **2** but only the at least one connector **26** of the at least one wire guide system. This embodiment prevents any risk of bad connections, saves time and gives sturdiness to the connection.

According to one embodiment, the electrode system **1** further comprises a power supply wire.

According to one embodiment, the electrode system **1** further comprises a battery. In this embodiment, the electrode system **1** is autonomous.

According to one embodiment, the power supply wire is layered to the at least one flexible band **11**.

According to one embodiment, the battery has an autonomy of 1h, 2h, 3h, 4h, 5h, 6h, 7h, 8h, 9h, 10h, 11h, 12h, 13h, 14h, 15h, 16h, 17h, 18h, 19h, 20h, 21h, 22h, 23h, 24h, 48h, 72h, 96h, 120h.

According to one embodiment, the battery is located on the at least one flexible band **11**.

According to one embodiment, the battery is positioned at a location of the electrode system **1** that allow the subject to be lying down comfortably. In this embodiment, the battery is not positioned at the back of the head of the subject.

According to one embodiment, the electrode system **1** further comprises at least one supplementary sensor.

According to one embodiment, the at least one supplementary sensor is configured for configured for measuring muscular, cardiac, ocular activity for improving the signal acquisition by discriminating different physiological artifacts from the skin voltage.

According to one embodiment, the at least one supplementary sensor is attached to the at least one flexible band **11**.

According to one embodiment, the at least one supplementary sensor is sewn or glued to the at least one flexible band **11**.

According to one embodiment, the at least one supplementary sensor is located on the at least one fitting means.

According to one embodiment, the at least one supplementary sensor is sewn or glued to the at least one fitting means.

According to one embodiment, the at least one supplementary sensor is located on the at least one fitting means at the jugular level.

According to one embodiment, the at least one supplementary sensor is an electromyogram sensor configured to detect an electromyogram signal simultaneously with the electrodes detecting at least one bio-signal.

According to one embodiment, the electromyogram signal is subtracted from the at least one bio-signal detected by the electrodes.

According to one embodiment, the electromyogram signal is a parasternal electromyogram signal.

According to one embodiment, the at least one supplementary sensor is an accelerometer. In this embodiment, the linear acceleration of the subject is acquired simultaneously with the signals from the at least one movable electrode module **13**.

According to one embodiment, the electrode system **1** further comprises a bonnet.

According to one embodiment, the bonnet comprises a plastic or an elastomer, such as for example polyamide, or acrylonitrile butadiene styrene.

According to one embodiment, the electrode system **1** further comprises at least one frontal band comprising a lower part and an upper part configured to couple together, a sealing ring, at least one orifice, at least one opening configured to receive at least one electrode. In this embodiment, the at least one frontal band replaces the at least one frontal electrode module. The at least one opening comprises a sealing ring to ensure the waterproofness of the at least one frontal band, at least one conductive cable **14** extends through the orifice, and said at least one frontal band comprises means for fastening the at least one electrode described hereabove.

According to one embodiment, the at least one frontal band further comprises means for detaching the at least one electrode from said at least one frontal band. In this embodiment, the means for detaching the at least one electrode is for example a textile band located between the at least one electrode and the at least one frontal band so that, by pulling the means for detaching the at least one electrode, said electrode is easily detached from the at least one frontal band.

According to one embodiment, the at least one frontal band is attached to the at least one first band **15**.

According to one embodiment, the at least one frontal band is attached to the at least one first band **15** using a spring rod.

According to one embodiment, the electrode system **1** is connected to an external device configured to process and analyze the bio-signal.

According to one embodiment, the external device is a computer, a smartphone, or a tablet computer.

According to one embodiment, the electrode system **1** is connected to the external device using a wired connection.

According to one embodiment, the electrode system **1** is connected to the external device using a wireless connection such as for example Bluetooth connection.

According to one embodiment, the external device is also configured to display a bio-signal.

According to one embodiment, the external device is also configured to display information relating to the positioning of the electrodes.

According to one embodiment, the electrode system **1** is further connected to a bio-signal processor for analyzing and interpreting the measured bio-signal.

According to one embodiment, the electrode system **1** is connected to the processor using a wired connection.

According to one embodiment, the electrode system **1** is connected to the processor using a wireless connection such as for example Bluetooth connection.

According to one embodiment, the processor for interpreting/analyzing bio-signals measured by means of the at least one electrode is located in the at least one control unit **2**, and is a chip.

According to one embodiment, the processor is an external processor for interpreting/analyzing bio-signal measured by means of the at least one electrode.

According to one embodiment, the external processor is located for example in the external device.

According to one embodiment, said external processor is remotely connected to the electrode system **1**, for example by means of a wireless transmitter and/or receiver.

According to one embodiment, said external processor is physically connected to the electrode system **1**, for example by means of a cable.

According to one embodiment, the external processor is a program or software such as Neuroscan, BioSemi, G-tech, Brain products or any equivalent software for monitoring an EEG signal. The processor may also include a software or a program for interpreting at least one bio-signal (for example an EEG, ECG) in order to correlate said at least one bio-signal with a particular mental state.

According to one embodiment, the electrode system **1** also comprises a memory for storing data related to the bio-signals.

According to one embodiment, the bio-signals detected by the at least one electrode are fed through a sensor interface and digitalized to be stored for subsequent processing.

*ELECTRODES SYSTEM 1'*

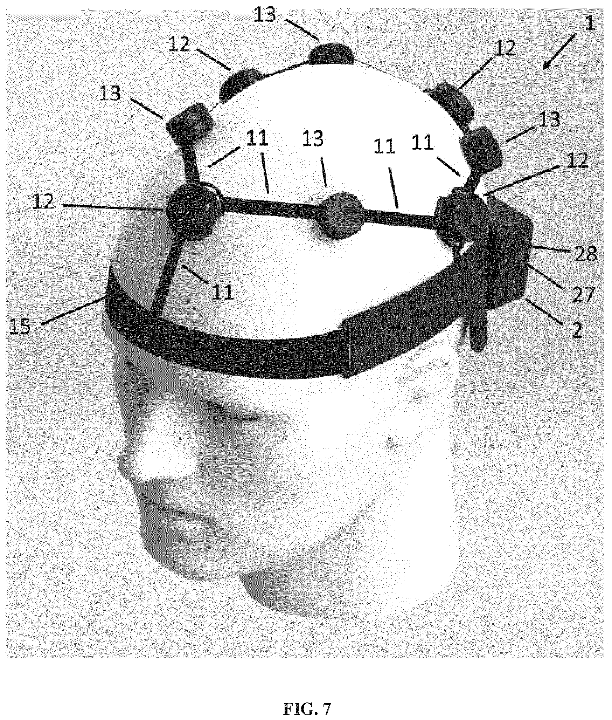

As illustrated on figure 7, the electrode system **1** further comprises at least two non-movable electrode modules **12** positionable at different locations on the at least one flexible band **11**, wherein the at least two non-movable electrode modules are fixed on said at least one flexible band.

According to one embodiment, the at least one flexible band **11** is the at least one flexible band **11** as described here above.

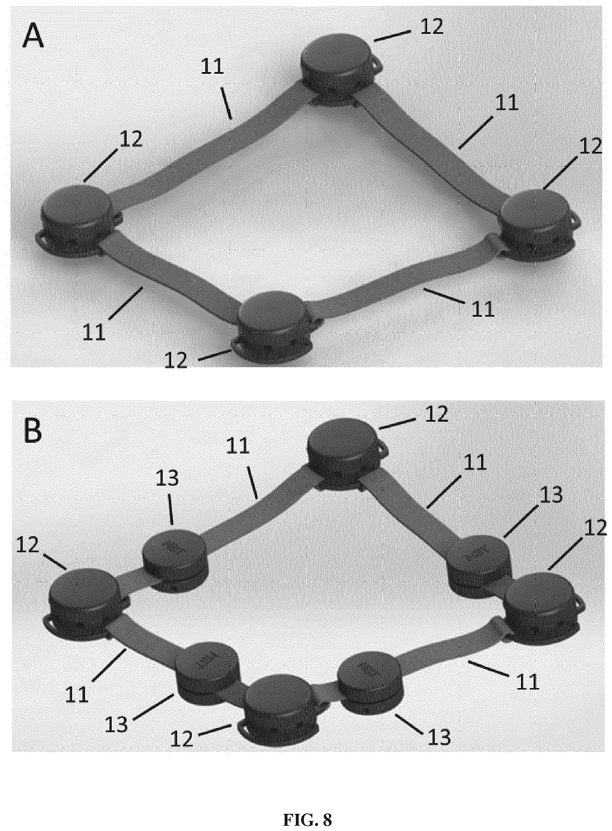

According to one embodiment, illustrated on figure 8A-B, the at least two non-movable electrode modules **12** define the network nodes of the flexible bands network.

According to one embodiment, illustrated on figure 8B, the at least one movable electrode module **13** is supported on at least one flexible band **11** between two network nodes.

The at least two non-movable electrode modules **12** defining the network nodes of the flexible bands network allow the flexible bands **11** to be steadily held, and the pressure of the flexible bands network to be equally divided on each measuring points.

According to one embodiment, the electrode system **1** comprises at least one, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, or 130 non-movable electrode modules **12**.

In a preferred embodiment, the electrode system **1** comprises 8, 16, 32, 64 or 128 non-movable electrode modules **12**.

According to one embodiment, the electrode system **1** comprises at least one empty electrode module. In this embodiment, at least one electrode module does not receive an electrode, said empty electrode module will enhance the contact and the secured positioning of the electrode system **1** on the head of the subject.

According to one embodiment, the at least two non-movable electrode modules **12** are removably positioned on the at least one flexible band **11**.

According to one embodiment, the at least two non-movable electrode modules **12** are disposable. In this embodiment, the disposability ensures a good hygiene and saves time.

According to one embodiment, the at least two non-movable electrode modules **12** have a round, a rectangular, a triangular, a polygon shape, or any other shape.

According to one embodiment, the at least two non-movable electrode modules **12** have a flat surface.

According to one embodiment, the at least two non-movable electrode modules **12** have a curved surface.

According to one embodiment, the at least two non-movable electrode modules **12** are disinfectable. In this embodiment, the at least two non-movable electrode modules **12** are disinfected using a cleaning bath, a disinfectant wipe, or any other means known by one skilled in the art.

According to one embodiment, the at least two non-movable electrode modules **12** comprise a lower part **121** and an upper part **122** configured to couple together.

According to one embodiment, the lower part **121** and the upper part **122** of the at least two non-movable electrode modules **12** couple together by screwing, or by using at least one clip.

According to one embodiment, the lower part **121** and the upper part **122** of the at least two non-movable electrode modules **12** comprise a plastic or an elastomer, such as for example polyamide, or acrylonitrile butadiene styrene.

According to one embodiment, the lower part **121** and the upper part **122** of the at least two non-movable electrode modules **12** comprise a biocompatible material. In this embodiment, the at least two non-movable electrode modules **12** are appropriate for a medical use, especially in a hospital environment.

According to one embodiment, the biocompatible material is certified to the requirements of USP Class VI.

According to one embodiment, the at least two non-movable electrode modules **12** comprise means for receiving and fastening the at least one electrode **126**.

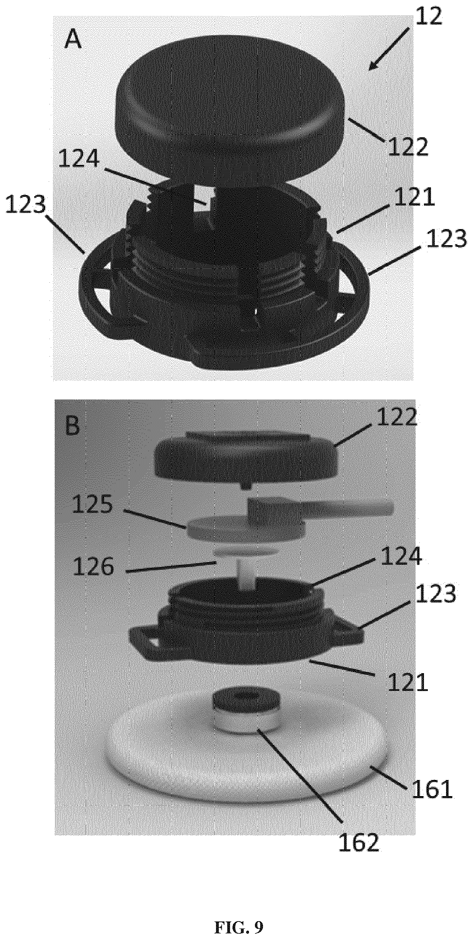

According to one embodiment, as illustrated on figure **9B**, the means for receiving and fastening the at least one electrode **126** is a snap fastener, or a locking groove.

According to one embodiment, the means for receiving and fastening the at least one electrode **126** is a female or a male snap fastener.

According to one embodiment, at least one extremity of the at least one conductive cable **14** is welded to the snap fastener. In this embodiment, the connection between the at least one conductive cable **14** and the at least one electrode is robust.

According to one embodiment, as illustrated on figure 9A, the at least two non-movable electrode modules **12** comprise a lower part **121** having a central opening and an upper part **122** configured to couple together, at least one attachment means **123** on which at least one flexible band **11** is attached, an orifice **124**, and at least one electrical connecting means **125**, wherein at least one conductive cable **14** extends through the orifice **124**, the lower part **121** is configured to receive at least one electrode through the central opening, and the at least one electrical connecting means **125** is configured to be connected to the at least one conductive cable **14** and to the at least one electrode.

According to one embodiment, at least one extremity of the at least one conductive cable **14** is welded to the at least one electrical connecting means. In this embodiment, the connection between the at least one conductive cable **14** and the at least one electrode is robust.

According to one embodiment, the at least one attachment means **123** is a handle for attachment on the at least one flexible band **11**.

According to one embodiment, the at least one attachment means **123** is at least two handles on which at least two flexible bands **11** can be attached.

According to one embodiment, the at least one attachment means **123** is two diametrically opposed handles on which at least two flexible bands **11** can be attached.

According to one embodiment, the at least one attachment means **123** is two diametrically opposed handles and at least one third handle on which at least three flexible bands **11** can be attached.

According to one embodiment, the at least one attachment means **123** is at least three handles on which at least three flexible bands **11** can be attached. In this embodiment, pressure from the flexible bands **11** is equally distributed on said at least one non-movable electrode module **12**.

According to one embodiment, the at least one flexible band **11** attached to the at least one attachment means **123** is sewn, glued, or attached with a self-gripping band to said at least one attachment means **123**.

According to one embodiment, the at least one electrical connecting means **125** comprises at least one printed circuit board (PCB). In this embodiment, the at least two non-movable electrode modules **12** each carries the electronics specific to the at least one electrode.

According to one embodiment, the at least one PCB is configured to receive and process a raw signal from the at least one electrode and to provide an output signal.

According to one embodiment, the at least one PCB is located on the wall, in the lower part **121** or in the upper part **122** of the at least two non-movable electrode modules **12**.

According to one embodiment, the at least one PCB comprises at least one connector to connect the at least one electrode.

According to one embodiment, the at least two non-movable electrode modules **12** comprise an insulating and waterproof silicone gel to ensure the waterproofness of said at least two non-movable electrode modules **12**.

According to one embodiment, the central opening comprises a flat seal.

According to one embodiment, the flat seal is made of rubber.

According to one embodiment, the at least two non-movable electrode modules **12** have a height ranging from 1 to 50 mm.

According to one embodiment, the height of the at least two non-movable electrode modules **12** is preferably 12 mm.

According to one embodiment, the at least two non-movable electrode modules **12** have a diameter ranging from 1 to 50 mm.

According to one embodiment, the diameter of the at least two non-movable electrode modules **12** is preferably 18 mm.

According to one embodiment, the at least two non-movable electrode modules **12** comprise at least one non-movable frontal electrode module and at least one non-movable cranial electrode module.

According to one embodiment, the at least one non-movable frontal electrode module is configured to receive at least one frontal electrode **16**.

According to one embodiment, the at least one non-movable cranial electrode module is configured to receive at least one cranial electrode **17**.

According to one embodiment, the at least one non-movable frontal electrode module has a height inferior to the height of the at least one non-movable cranial electrode module. In this embodiment, its smaller height makes the at least one non-movable frontal electrode module hardly visible in the electrode system **1**.

According to one embodiment, the central opening of the at least one non-movable cranial electrode module comprises a number of through holes equal to the number of pins **172**. In this embodiment, each pin **172** of the at least one cranial electrode **17** exits the at least one non-movable cranial electrode module by one of the said through holes.

According to one embodiment, the at least one non-movable frontal electrode module has a height inferior to the height of the at least one movable electrode module **13**. In this embodiment, its smaller height makes the at least one non-movable frontal electrode module hardly visible in the electrode system **1**.

According to one embodiment, the electrode system **1** comprises at least one empty electrode module **12**. In this embodiment, at least one empty electrode module does not receive an electrode, said empty electrode module will enhance the contact and the secured positioning of the electrode system **1** on the head of the subject.

According to one embodiment, the at least one conductive cable **14** passes through the orifice **124** of the at least two non-movable electrode modules **12** in order to go through said at least one non-movable electrode module **12** to reach the next electrode module **12**.

According to one embodiment, the at least one conductive cable **14** is connected to at least one non-movable electrode module **12** and layered against the flexible band **11** supporting said at least one non-movable electrode module **12**. In this embodiment, the length of the at least one conductive cable **14** is equal to the maximal stretched length of said flexible band **11**.

According to one embodiment, at least one extremity of the at least one conductive cable **14** is welded to at least one non-movable electrode module **12**. In this embodiment, the at least one conductive cable **14** is connected to the at least one electrode upon reception of said at least one electrode in the corresponding non-movable electrode module **12**.

According to one embodiment, at least one extremity of the at least one conductive cable **14** is welded to the at least one electrical connecting means **125** of at least one non-movable electrode module **12**.

According to one embodiment, the at least two non-movable electrode modules **12** and the at least one electrode are disposable. In this embodiment, the disposability ensures a good hygiene and saves time.

According to one embodiment, the at least one electrode comprises a female snap fastener and the at least two non-movable electrode modules **12** comprise a male snap fastener. In this embodiment, the at least one electrode is easily fastened in the at least two non-movable electrode modules **13**, and the overload of the electrode system **1** is kept to a minimum.

According to one embodiment, the at least one electrode comprises a male snap fastener and the at least two non-movable electrode modules **12** comprise a female snap fastener. In this embodiment, the at least one electrode is easily fastened in the at least two non-movable electrode modules **12**, and the overload of the electrode system **1** is kept to a minimum.

According to one embodiment, the at least two non-movable electrode modules **12** comprises at least one locking groove and the upper part of the at least one electrode comprises at least one projection configured to fit in said locking groove to fasten the at least one electrode in the at least two non-movable electrode modules **12**.

According to one embodiment, the lower part of the at least one electrode comprises at least one recess and the at least two non-movable electrode modules **12** comprises at least one projection configured to fit in said recess. In this embodiment, coupling of the projection in the recess ensure alignment of the connectors of the at least one electrode and the at least two non-movable electrode modules **12**.

According to one embodiment, the at least one electronic control unit **2** comprises means for controlling the recording of signals from the at least two non-movable electrode modules **12**.

According to one embodiment, the means for controlling the recording of signals from the at least two non-movable electrode modules **12** is a button or a switch, preferably a O/I switch.

According to one embodiment, the means for controlling the recording of signals from the at least two non-movable electrode modules **12** is of different color compared to the electronic control unit **2**.

According to one embodiment, the means for switching on the at least one electronic control unit **27** and the means for controlling the recording of signals from the at least two non-movable electrode modules **12** are one and the same.

According to one embodiment, the at least one supplementary sensor is an accelerometer. In this embodiment, the linear acceleration of the subject is acquired simultaneously with the signals from the at least two non-movable electrode modules **12**.

*ELECTRODES SYSTEM 2*

The present invention also relates to an electrode system **1**.
The electrode system **1** comprises:

- at least one flexible band **11**;
- at least one electrode module **13** comprising a lower part **131** and an upper part **132** configured to couple in a first position and in a second position;

wherein the at least one flexible band **11** passes between said lower **131** and upper part **132** of the at least one electrode module **13**, and is retained in the first position or free to move in the second position.
According to one embodiment, the at least one flexible band **11** is the at least one flexible band **11** as described here above.
According to one embodiment, the at least one electrode module is the electrode module as described here above.
According to one embodiment, the electrode system **1** comprises at least one empty electrode module. In this embodiment, at least one empty electrode module does not receive an electrode, said empty electrode module will enhance the contact and the secured positioning of the electrode system **1** on the head of the subject.
According to one embodiment, the at least one conductive cable **14** is the at least one conductive cable **14** as described here above.
According to one embodiment, the electrode system **1** is configured to detect at least one bio-signal of a subject.
According to one embodiment, the electrode system **1** is configured to detect brain signals such as for example EEG signals of a subject.
According to one embodiment, the electrode system **1** is a headgear, a helmet or a hat.
According to one embodiment, the electrode system **1** is disinfectable. In this embodiment, the electrode system **1** is disinfected using a cleaning bath, a disinfectant wipe, or any other means known by one skilled in the art.
According to one embodiment, the electrode system **1** is disposable. In this embodiment, the disposability ensures a good hygiene and saves time.
According to one embodiment, the electrode system **1** is designed for clinical or non-clinical application.
According to one embodiment, the electrode system **1** comprises at least one electrode as described here above.
According to one embodiment, the electrode system **1** comprises a wireless transmitter/transceiver.
According to one embodiment, the electrode system **1** further comprises at least one hoop as described here above.
According to one embodiment, the electrode system **1** further comprises at least one fitting means as described here above.
According to one embodiment, the electrode system **1** further comprises at least one electronic control unit **2** as described here above.
According to one embodiment, the electrode system **1** further comprises a power supply wire as described here above. According to one embodiment, the electrode system **1** further comprises a battery as described here above.
According to one embodiment, the electrode system **1** further comprises at least one supplementary sensor as described here above.
According to one embodiment, the electrode system **1** further comprises a bonnet as described here above.
According to one embodiment, the electrode system **1** further comprises at least one frontal band as described here above.

*ELECTRODE SYSTEM ADJUSTMENT*

The present invention also relates to a method for adjusting the electrode system **1** of the invention.
The method for adjusting the electrode system **1** of the invention comprises the following steps:

- calculating the distance between each measurement points according to a measuring system 10/20, 10/10, or 10/5, and depending on a head size model;
- determining the number and position of wished measurement points for the acquisition of a signal with the electrode system **1** of the invention; and
- calculating the length of the flexible bands **11** between each wished measurement point, and the distance between the flexible bands **11** depending on the electrode module diameter and the flexible bands **11** stretching.

According to one embodiment, there are at least two head size models labeled small or medium.

According to one embodiment, there are at least three head size models labeled small, medium, or large. In this embodiment, choosing an appropriate head size model for a subject allows a precise adjustment of the electrode system **1** of the invention.

*PRE-PROCESSING METHOD*

The present invention also relates to a method for pre-processing a signal acquired with the electrode system **1** of the invention.

The method for pre-processing a signal comprises the following steps:

- increasing the common-mode rejection ratio (CMRR);
- decreasing the signal dynamics to prevent saturation;
- filtering the DC component of said signal;
- filtering low frequencies; and
- filtering high frequencies to prevent spectrum folding.

The use of dry electrodes, especially different types of dry electrodes (spring-loaded and textile), requires for a method for pre-processing a signal to make up for impedance differences between said types of electrodes or even between several electrodes of same type used on wet or dry skin. The steps described hereabove improve the signal-to-noise ratio, thus enabling signal to be observed.

According to one embodiment, the signal is a bio-signal.

According to one embodiment, the signal is a EEG signal.

According to one embodiment, an analog processing stage implementing the method for pre-processing a signal is added upstream of the at least one control unit **2** described hereabove.

According to one embodiment, an analog processing stage implementing the method for pre-processing a signal comprises at least one low pass filter and at least one high input impedance following circuit placed after said at least one electrode.

According to one embodiment, an analog processing stage implementing the method for pre-processing a signal comprises, after the at least one electrode, an impedance transformer (unity gain amplifier), a high pass filter and a low to receive at least one means for connecting at least two electronic control pass filter.

According to one embodiment, the low frequencies comprises motion artefacts and/or variations of galvanic voltages.

*SYNCHRONIZATION SYSTEM*

The present invention also relates to a system for synchronizing a signal acquired with the electrode system **1** of the invention and at least one external stimulus.

The system for synchronizing a signal acquired with the electrode system **1** of the invention and at least one external stimulus comprises:

- a housing;
- at least one power supply;
- at least one demodulating means;
- at least one amplifying means; and
- at least one socket.

The Evoked Response Potential (ERP) test refers to the modification of the electrical potential produced by the nervous system in response to an external stimulus, in particular a sensory stimulus (audio, or video for example). Since the measured electrical signals are generally very low, it is often necessary to repeat the recording a great number of times to average all the measures in order to increase the signal-to-noise ratio and to obtain a reliable ERP. Therefore, the measurement of the ERP requires to know precisely the time at which the stimulus was transmitted, and then to superimpose it temporally with the electrical signal acquired. The system for synchronizing a signal acquired with the electrode system **1** of the invention and at least one external stimulus allows the synchronized acquisition of a signal with the electrode system **1** of the invention and at least one external stimulus. Thus, it allows an easy and fast measurement of the ERP.

In case of an audio stimulus, the audio signal transmitted is stereo, it carries on its right channel the audio stimulus to be listened to by the subject, and on its left channel the synchronized audio signal positioned at desired instants of the recording. The synchronized audio signal is an amplitude modulation, it is demodulated to condition it into a signal usable by the electrode system **1** of the invention. The latter then superimposes the synchronized audio signal on the signal acquired with the electrode system **1** of the invention.

According to one embodiment, the at least one external stimulus is an audio stimulus, a visual stimulus, or a mixture thereof.

According to one embodiment, the at least one power supply is a battery.

According to one embodiment, the housing comprises a lower part and an upper part configured to couple together by gluing, screwing, or means of at least one clamping clip.

According to one embodiment, the system for synchronizing a signal acquired with the electrode system **1** of the invention and at least one external stimulus is connected to the electrode system **1** of the invention by the at least one socket.

According to one embodiment, the system for synchronizing a signal acquired with the electrode system **1** of the invention and at least one external stimulus is connected to a smartphone, a computer, or a tablet by the at least one socket.

According to one embodiment, the system for synchronizing a signal acquired with the electrode system **1** of the invention and at least one external stimulus is connected to means for receiving the at least one external stimulus by the at least one socket.

According to one embodiment, the means for receiving the at least one external stimulus comprises an audio system such as headphones for example, a video system such as a screen for example, or a mixture thereof.

According to one embodiment, the system for synchronizing a signal acquired with the electrode system **1** of the invention and at least one external stimulus is connected to the electrode system **1** of the invention and a smartphone, a computer, or a tablet.

According to one embodiment, the system for synchronizing a signal acquired with the electrode system **1** of the invention and at least one external stimulus is connected to the electrode system **1** of the invention and means for receiving the at least one external stimulus.

According to one embodiment, the system for synchronizing a signal acquired with the electrode system **1** of the invention and at least one external stimulus is connected to a smartphone, a computer, or a tablet and means for receiving the at least one external stimulus.

According to one embodiment, the system for synchronizing a signal acquired with the electrode system **1** of the invention and at least one external stimulus is connected to the electrode system **1** of the invention; a smartphone, a computer, or a tablet; and means for receiving the at least one external stimulus.

According to one embodiment, the at least one socket is a jack socket, preferably a mini jack socket.

According to one embodiment, the system for synchronizing a signal acquired with the electrode system **1** of the invention and at least one external stimulus comprises at least two sockets.

According to one embodiment, the system for synchronizing a signal acquired with the electrode system **1** of the invention and at least one external stimulus comprises at least three sockets. In this embodiment, the system for synchronizing a signal acquired with the electrode system **1** of the invention and at least one external stimulus can be connected to the electrode system **1** of the invention; a smartphone, a computer, or a tablet; and means for receiving the at least one external stimulus.

According to one embodiment, the system for synchronizing a signal acquired with the electrode system **1** of the invention and at least one external stimulus comprises at least one printed circuit board comprising the at least the at least one demodulating means, and the at least one amplifying means.

According to one embodiment, the system for synchronizing a signal acquired with the electrode system **1** of the invention and at least one external stimulus further comprises means for recording the reaction of the subject upon receiving the at least one external stimulus. In this embodiment, a voluntary and motor reaction of the subject to the at least one external stimulus is recorded, and a signal is then generated, allowing this supplementary information to be superimposed on the signal acquired with the electrode system **1** of the invention.

According to one embodiment, the means for recording the reaction of the subject upon receiving the at least one external stimulus is a push button.

According to one embodiment, the system for synchronizing a signal acquired with the electrode system **1** of the invention and at least one external stimulus is connected to at least two electrode systems **1** of the invention; a smartphone, a computer, or a tablet; and means for receiving the at least one external stimulus. In this embodiment, at least two subject receives simultaneously the same at least one external stimulus. This embodiment makes it possible to study the interactions between two subjects measured simultaneously, during social interactions for example.

According to one embodiment, the synchronized signal is superimposed to the at least one audio stimulus, and has a higher frequency than the at least one audio stimulus, high enough to be inaudible to the subject. In this embodiment, the at least one audio stimulus is a stereo signal.

While various embodiments have been described and illustrated, the detailed description is not to be construed as being limited hereto. Various modifications can be made to the embodiments by those skilled in the art without departing from the true spirit and scope of the disclosure as defined by the claims.

**BRIEF DESCRIPTION OF THE DRAWINGS**

**Figure 1** illustrates the electrode system **1** of the invention comprising a flexible bands network comprising a first band **15** and flexible bands **11** movable electrode modules **13**; and an electronic control unit **2** comprising means for switching on the electronic control unit **27** and a LED **28**.

**Figure 2** is a photograph showing a movable electrode module **13** in an open and closed configuration.

**Figure 2A** is a photograph showing a movable electrode module **13** in an open configuration, comprising an aperture **133** wherein the lower part **131** and the upper part **132** are not coupled together.

**Figure 2B** is a photograph showing a movable electrode module **13** in a closed configuration, comprising an aperture **133** wherein the lower part **131** and the upper part **132** are coupled together and one flexible band **11** extends through the aperture **133** of the movable electrode module **13.**

**Figure 3** shows dry electrodes.

**Figure 3A** shows a textile dry electrode comprising a foam coated with a conductive fabric **161** and a snap fastener **162.**

**Figure 3B** shows a textile dry electrode comprising a conductive fabric **163**, a support **164** and a relatively impermeable layer **165** in-between.

**Figure 3C** shows a textile dry electrode comprising a conductive fabric **163**, a support **164**, a relatively impermeable layer **165** in-between and an absorbent material **167**.

**Figure 3D** shows a spring-loaded dry electrode comprising 4 rows of 2 pins **172** and a housing **171**.

**Figure 4** illustrates different shapes of a cranial electrode **17** comprising a PCB **173** and a plurality of pins **172**.

**Figure 4A** illustrates a cranial electrode **17** comprising a PCB **173** and a plurality of pins **172**.

**Figure 4B** illustrates a cranial electrode **17** comprising a PCB **173** and a plurality of pins **172** in an electrode module **13.**

**Figure 4C** illustrates a cranial electrode **17** comprising a PCB **173** and a plurality of pins **172**.

**Figure 4D** illustrates a cranial electrode **17** comprising a PCB **173** and a plurality of pins **172** in an electrode module **13.**

**Figure 4E** illustrates a cranial electrode **17** comprising a PCB **173** and a plurality of pins **172**.

**Figure 4F** illustrates a cranial electrode **17** comprising a PCB **173** and a plurality of pins **172** in an electrode module **13.**

**Figure 5** illustrates a conductive cable **14** in a relaxed and a stretched configuration.

**Figure 5A** shows a conductive cable **14** layered against a flexible band **11** using textile rings **18** and connected to an electronic control unit **2** and a spring-loaded electrode **17**, wherein the electrode system **1** is in a relaxed configuration.

**Figure 5B** shows a conductive cable **14** layered against a flexible band **11** using textile rings **18** and connected to an electronic control unit **2** and a spring-loaded electrode **17**, wherein the electrode system **1** is in a stretched configuration.

**Figure 6** is an exploded view of an electronic control unit **2** comprising a lower part **21**, an upper part **22**, a MCU **23**, a battery **24**, a O/I switch, a LED **28**, a jack socket **25**, and a connector **26**.

**Figure 7** illustrates the electrode system **1** of the invention comprising a flexible bands network comprising a first band **15** and flexible bands **11**; non-movable electrode modules **12**; movable electrode modules **13**; and an electronic control unit **2** comprising means for switching on the electronic control unit and a LED.

**Figure 8** is a photograph showing the flexible bands network comprising flexible bands **11** and electrode modules (**12**, **13**).

**Figure 8A** is a photograph showing the flexible bands network comprising flexible bands **11** and non-movable electrode modules **12.**

**Figure 8B** is a photograph showing the flexible bands network comprising flexible bands **11**, non-movable electrode modules **12** and movable electrode modules **13**.

**Figure 9** illustrates a non-movable electrode module **12**.

**Figure 9A** is a photograph showing a non-movable electrode module **12** comprising a lower part **121**, an upper part **122**, means for attachment **123**, and an orifice **124**.

**Figure 9B** is a photograph showing a non-movable electrode module **12** comprising a lower part **121**, an upper part **122**, means for attachment **123**, an orifice **124**, an electrical connecting means **125**, and means for receiving and fastening the at least one electrode **126**, the at least one electrode comprising a foam coated with a conductive fabric 161 and a snap fastener **162**.

**Figure 10** represents the EEG power spectrum density of one subject recorded in two conditions at rest respectively with eyes closed (solid line) and then eyes opened (dot line).

## EXAMPLE

The present invention is further illustrated by the following example.

Example 1:

### *Materials and Methods*

*Material*

Electrode system **1** of the invention comprising:

- flexible bands (11) in polypropylene;
- electrode modules (12, 13) positionable at different locations on the flexible bands (11) in ABS (injection), or polyamid (powder sintering);
- conductive cables (14) connected to the electrode modules (12, 13);
- electrodes configured to fit in one electrode module (12, 13), to be connected with one conductive cable (14) and to detect at least one bio-signal; and
- electronic control unit (2).

The plastic parts of said electrode system **1** are made by injection-molding in case of ABS, or by powder sintering in case of polyamide.

### *Results*

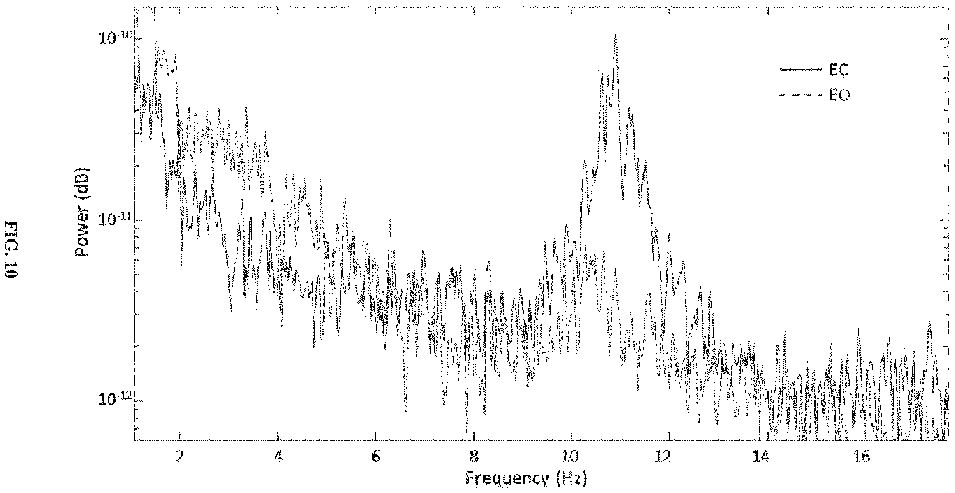

The figure 10 represents the EEG power spectrum density of one subject recorded in two conditions at rest respectively with eyes closed (solid line) and then eyes opened (dot line). A large modulation of alpha waves activity can be seen in the frequency band between 10 Hz and 12 Hz. Eyes closed condition imply an increase of alpha waves activity expected from the literature. This effect is knowing as "alpha blocking effect" due to the thalamo-cortical loop, which is a good marker EEG brain waves detection.

**REFERENCES**

1 - Electrode system;
11 - Flexible band;
12 - Non-movable electrode module;
121 - Lower part of a non-movable electrode module;
122 - Upper part of a non-movable electrode module;
123 - Attachment means;
124 - Orifice;
125 - Electrical connecting means;
126 - Means for receiving and fastening the at least one electrode;
13 - Movable electrode module;
131 - Lower part of a movable electrode module;
132 - Upper part of a movable electrode module;
133 - Aperture;
14 - Conductive cable;
15 - First band;
16 - Frontal electrode;
161 - Foam coated with a conductive fabric;
162 - Snap fastener;
163 - Conductive fabric;
164 - Support;
165 - Relatively impermeable layer;
166 - Means to be connected to the at least one electrical connecting means of one electrode module;
167 - Absorbent material;
17 - Cranial electrode;
171 - Housing;
172 - Pin;
173 - PCB;
18 - Textile ring;
2 - Electronic control unit;
21 - Lower part of the electronic control unit;
22 - Upper part of the electronic control unit;
23 - MCU;
24 - Battery;
25 - Socket;
26 - Connector;
27 - Means for switching on the electronic control unit;
28 - LED.

**Claims**

1. An electrode system (1) comprising:

   - at least one flexible band (11);
   - at least one movable electrode module (13) having at least one degree of freedom with reference to said at least one flexible band (11) and positionable at different locations on the at least one flexible band (11);
   - at least one conductive cable (14) connected to the at least one movable electrode module (13); and
   - at least one electrode configured to fit in one movable electrode module (13), to be connected with the at least one conductive cable (14) and to detect at least one bio-signal.

2. The electrode system (1) according to claim **1**, wherein said at least one movable electrode module (13) comprises a lower part (131) and an upper part (132) configured to couple together in a first position and in a second position, the at least one flexible band (11) passes between said lower (131) and upper (132) parts, and is retained in the first position or free to move in the second position.

3. The electrode system (1) according to claim **1** or claim **2**, wherein the at least one flexible band (11) is a network of flexible bands.

4. The electrode system (1) according to anyone of the preceding claims **1** to **3**, wherein the flexible bands network comprises at least one first band (15) configured to be placed around the skull and a network of at least one flexible band (11) with voids therebetween, attached to the at least one first band (15) and configured to ensure a link between the at least one first band (15) and the at least one movable electrode module (13).

5. The electrode system (1) according to anyone of the preceding claims **1** to **4**, wherein the at least one movable electrode module (13) comprises a lower part (131) having a central opening and an upper part (132) configured to couple together, at least one orifice, at least one electrical connecting means, and at least one aperture (133) between said lower (131) and upper (132) parts, wherein at least one conductive cable (14) extends through the orifice, the lower part (131) is configured to receive at least one electrode through the central opening, the at least one electrical connecting means is configured to be connected to the at least one conductive cable (14) and to the at least one electrode, and at least one flexible band (11) extends through the at least one aperture (133) so that the at least one movable electrode module (13) can move along said flexible band (11).

6. The electrode system (1) according to anyone of the preceding claims **1** to **5**, wherein the at least one electrode is removable.

7. The electrode system (1) according to anyone of the preceding claims **1** to **6**, wherein the at least one electrode is a dry electrode.

8. The electrode system (1) according to anyone of the preceding claims **1** to **7**, wherein the at least one conductive cable (14) is layered against the at least one flexible band (11), preferably the at least one conductive cable (14) is layered against the at least one flexible band (11) using at least one textile ring (18).

9. The electrode system (1) according to anyone of the preceding claims **1** to **8**, wherein the flexible bands network is configured to be articulated in the form of a network according to the measuring system 10/20.

10. The electrode system (1) according to anyone of the preceding claims **1** to **9**, wherein the electrode system (1) is a headgear.

11. The electrode system (1) according to anyone of the preceding claims **1** to **10**, wherein the electrode system (1) further comprises at least one electronic control unit (2) comprising at least one of the following functional parts:

   - an A/D interface;
   - a signal processing part;
   - a power supply;
   - a wire communication part and/or a wireless communication part; and
   - a micro-controller unit (MCU) (23);

wherein said at least one control unit (2) is connected to the at least one electrode by the at least one conductive cable (14).

12. The electrode system (1) according to anyone of the preceding claims **1** to **11**, wherein the electrode system (1) further comprises at least two non-movable electrode modules (12) positionable at different locations on the at least one flexible band (11), wherein the at least two non-movable electrode modules are fixed on said at least one flexible band, said at least two non-movable electrode modules (12) define the network nodes of the flexible bands network, and the at least one movable electrode module (13) is supported on at least one flexible band (11) between two network nodes.

13. The electrode system (1) according to claim **12**, wherein the at least two non-movable electrode modules (12) comprise a lower part (121) having a central opening and an upper part (122) configured to couple together, at least one attachment means (123) on which at least one flexible band (11) is attached, an orifice (124), and at least one electrical connecting means (125), wherein at least one conductive cable (14) extends through the orifice (124), the lower part (121) is configured to receive at least one electrode through the central opening, and the at least one electrical connecting means (125) is configured to be connected to the at least one conductive cable (14) and to the at least one electrode.

14. The electrode system (1) according to claim **12** or claim **13**, wherein the at least two non-movable electrode modules (12) comprise at least one non-movable frontal electrode module configured to receive at least one frontal electrode (16) and at least one non-movable cranial electrode module configured to receive at least one cranial electrode (17), wherein the at least one frontal electrode (16) is configured to be in contact with the frontal skin of the subject when in the at least one frontal electrode module, and comprises a foam and conductive silver fibers; and the at least one cranial electrode (17) is configured to be in contact with the skin of the subject through the hair when in the at least one cranial electrode module, and comprises a housing (171) and a plurality of spring mounted pins (172).

15. An electrode system (1) comprising:

- at least one flexible band (11); and
- at least one electrode module (13) comprising a lower part (131) and an upper part (132) configured to couple in a first position and in a second position;

wherein the at least one flexible band (11) passes between said lower (131) and upper (132) parts of at least one electrode module (13), and is retained in the first position or free to move in the second position.

**FIG. 1**

FIG. 2

FIG. 3

FIG. 4

FIG. 5

FIG. 6

FIG. 7

FIG. 8

**FIG. 9**

**FIG. 10**

Europäisches
Patentamt

European
Patent Office

Office européen
des brevets

# EUROPEAN SEARCH REPORT

Application Number

EP 17 15 9382

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GB 2 516 275 A (INVEST CH AG W [CH]) 21 January 2015 (2015-01-21) * abstract * * page 4, line 28 - page 8, line 20 * ----- | 1-15 | INV. A61B5/0478 A61B5/00 |
| X | US 2012/143020 A1 (BORDOLEY MORDECHAI [US] ET AL) 7 June 2012 (2012-06-07) * paragraph [0138] - paragraph [0147] * * figures 3, 4 * ----- | 1-15 | |
| A | US 2001/044573 A1 (MANOLI SAMIR [US] ET AL) 22 November 2001 (2001-11-22) * the whole document * ----- | 1-15 | |

TECHNICAL FIELDS
SEARCHED      (IPC)

A61B

The present search report has been drawn up for all claims

| Place of search | Date of completion of the search | Examiner |
|---|---|---|
| The Hague | 24 August 2017 | Van Dop, Erik |

EPO FORM 1503 03.82 (P04C01)

## ANNEX TO THE EUROPEAN SEARCH REPORT
## ON EUROPEAN PATENT APPLICATION NO.

EP 17 15 9382

This annex lists the patent family members relating to the patent documents cited in the above-mentioned European search report.
The members are as contained in the European Patent Office EDP file on
The European Patent Office is in no way liable for these particulars which are merely given for the purpose of information.

24-08-2017

| Patent document cited in search report | | Publication date | Patent family member(s) | | Publication date |
|---|---|---|---|---|---|
| GB 2516275 | A | 21-01-2015 | NONE | | |
| US 2012143020 | A1 | 07-06-2012 | NONE | | |
| US 2001044573 | A1 | 22-11-2001 | AU | 2002320527 A1 | 29-01-2003 |
| | | | US | 2001044573 A1 | 22-11-2001 |
| | | | WO | 03005897 A2 | 23-01-2003 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2007225585 A

- US 8463354 B